# EUROPEAN PATENT APPLICATION

(11) **EP 4 844 057 A1**
(43) Date of publication of application: **25.11.2026**
(21) Application number: 25751605.4
(22) Date of filing: 06.02.2025
(51) Int. Cl.: C12N 5/10, C12N 15/62, A61K 39/00, A61P 35/00, C07K 19/00, A61K 35/17

(54) **ENGINEERED CELL AND USE THEREOF**

(30) Priority: 06.02.2024 WO PCT/CN2024/076386
(71) Applicant: Imvivabio LLC, Boston, MA 02108 (US)
(72) Inventor: ZHOU, Yali, Hong Kong 999077 (HK); CHEN, Gong, Hong Kong 999077 (HK); ZENG, Jiuqin, Hong Kong 999077 (HK); REN, Jiangtao, Hong Kong 999077 (HK)
(74) Representative: Comoglio, Elena
(86) International application number: PCT/CN2025/076047
(87) International publication number: WO 2025/168006

(57) **Abstract**

The present invention relates to an engineered cell which expresses (i) a PD1-binding molecule comprising a PD1-binding domain and a transmembrane domain and not comprising any intracellular domain other than an intracellular domain derived from a PD1 ligand; (ii) an immunosuppressive molecule comprising an immunosuppressive protein binding domain and a transmembrane domain and not comprising a primary signaling domain, wherein the immunosuppressive protein is selected from NKG2A, TIM3, LAG3, TIGIT, CTLA4, IRP60, SIRPα, KIR2DL1/2/3, LILRB1 or a combination thereof. The present invention also relates to a composition comprising the engineered cell and use of the engineered cell.

## Description

### CROSS-REFERENCE TO RELATED APPLICATION

The present application claims the priority to the PCT international application with the international application No. PCT/CN2024/076386, and entitled "ENGINEERED CELL AND USE THEREOF", filed on February 06, 2024, which is incorporated in the present disclosure by reference in its entirety.

### TECHNICAL FIELD

The present invention pertains to the field of cell therapy. More specifically, the present invention relates to an engineered cell expressing a PD1-binding molecule and an immunosuppressive molecule, and use thereof.

### BACKGROUND ART

As an emerging immunotherapy, adoptive cell therapy has developed very rapidly in recent years. Particularly for chimeric antigen receptor therapy (CAR-T therapy), many products have been launched and have shown excellent therapeutic effects in clinical practice. However, while CAR-T therapy exhibits significant efficacy, it is also accompanied by various adverse reactions, including cytokine release syndrome (CRS, also known as cytokine storm), neurotoxicity, tumor lysis syndrome, hemocytopenia, infection, hypoimmunoglobulinemia, and hepatitis B virus reactivation, etc.

CRS refers to the massive release of cytokines during the interaction of immune cells and tumor cells; these cytokines trigger further chain reactions, such as excessive inflammatory response, capillary leakage, and coagulation cascades, further leading to organ damage and brain swelling. CRS is the most frequent and symptomatic acute toxicity reaction in CAR-T cell therapy. It has been reported that in clinical trials, over 90% of patients using anti-CD19 CAR-T cells to treat B-cell acute lymphoblastic leukemia (B-ALL) and non-Hodgkin's B-cell lymphoma experienced CRS. Therefore, it remains necessary to improve existing CAR-T therapies to reduce adverse reactions and achieve better therapeutic effects.

### SUMMARY

Unless otherwise specified, all scientific and technical terms used herein have the same meaning as commonly understood by one of ordinary skill in the art to which the present invention pertains.

In a first aspect, the present invention provides an engineered cell that expresses:
(i) a PD1-binding molecule comprising a PD1-binding domain and a transmembrane domain and not comprising any intracellular domain other than an intracellular domain derived from a PD1 ligand;
(ii) an immunosuppressive molecule comprising an immunosuppressive protein binding domain and a transmembrane domain and not comprising a primary signaling domain, wherein the immunosuppressive protein is selected from NKG2A, TIM3, LAG3, TIGIT, CTLA4, IRP60, SIRPα, KIR2DL1/2/3, LILRB1 or a combination thereof.

In some embodiments, the immunosuppressive molecule further comprises a co-stimulatory domain. In some other embodiments, the immunosuppressive molecule does not comprise a co-stimulatory domain.

### PD1-binding molecules and immunosuppressive molecules

The term "immunosuppressive molecule" refers to a molecule capable of binding to an immunosuppressive protein (e.g. NKG2A, TIM3, LAG3, TIGIT, CTLA4, IRP60, SIRPα, KIR2DL1/2/3, LILRB1, etc.) and in turn suppressing immune rejection of exogenous cells by a subject, for example, reducing the killing function of immune cells (e.g. T cells, NK cells, etc.) in the subject or suppressing the excessive proliferation of immune cells. The term "immunosuppressive protein" refers to a ligand or a receptor that exert a suppressive effect on immune response, which plays an important role in maintaining autoimmune tolerance and regulating the duration and magnitude of immune response, thereby preventing the immune system from damaging and destroying normal tissues. The immunosuppressive protein of the present invention is selected from NKG2A, TIM3, LAG3, TIGIT, CTLA4, IRP60, SIRPα, KIR2DL1/2/3, LILRB1 or a combination thereof.

The term "PD1 binding domain" refers to any structure (e.g. an antibody, a ligand, or a receptor, etc.) that can bind to PD1, or a functional variant of such structure. The term "immunosuppressive protein binding domain" refers to any structure (e.g. an antibody, a ligand, or a receptor, etc.) that can bind to an immunosuppressive protein or a functional variant thereof.

In some embodiments, the binding domain of the present invention is selected from an antibody. The term "antibody" has the broadest meaning as understood by those skilled in the art and includes intact antibodies, such as monoclonal antibodies, polyclonal antibodies, multivalent antibodies, and multispecific antibodies (e.g., bispecific antibodies), and antibody fragments or synthetic polypeptides carrying one or more CDR sequences capable of exhibiting the desired biological activity, which may be of any class (e.g., IgG, IgE, IgM, IgD, IgA, etc.) or subclass (e.g., IgG1, IgG2, IgG2a, IgG3, IgG4, IgA1, IgA2, etc.). The term "antibody fragment" refers to at least a moiety of an intact antibody or variant thereof, and refers to a binding domain (e.g., the antigen variable region of an intact antibody) sufficient to confer upon the antibody fragment the ability to recognize and specifically bind to a target (e.g., an antigen). Examples of the antibody fragment include, but are not limited to, Fab, Fab', F(ab')2, Fd fragment, Fd', Fv fragment, scFv, disulfide-linked Fv (sdFv), linear antibody, "diabody" with two antigen binding sites, single domain antibody (sdAb) (e.g., heavy chain variable region VH of an antibody, light chain variable region VL, nanobody VHH, etc.).

In some embodiments, the "PD1 binding domain" of the present invention is selected from a ligand, a receptor that binds to PD1 and a functional fragment thereof (i.e., a functional fragment with PD1 binding ability, e.g. an extracellular region). In an embodiment, the PD1-binding domain and the transmembrane domain comprised in the PD1-binding molecule are derived from the same molecule or from different molecules, and do not comprise any intracellular domains (e.g. co-stimulatory domains and/or primary signaling domains). In another embodiment, the PD1-binding domain and the transmembrane domain comprised in the PD1-binding molecule are derived from the same molecule or from different molecules, and comprise an intracellular domain derived from a PD1 ligand. In other words, the PD1-binding molecule of the present invention may be the PD1 ligand itself.

In some embodiments, the "immunosuppressive protein binding domain" of the present invention is selected from a ligand, a receptor that binds to the immunosuppressive protein and a functional fragment thereof (i.e., a functional fragment having immunosuppressive protein binding ability, e.g. an extracellular region). In an embodiment, the three domains comprised in the immunosuppressive molecule, namely the immunosuppressive protein binding domain, the transmembrane domain and the co-stimulatory domain, are not all derived from the same molecule. For example, two of the domains are derived from the same molecule, while the other domain is derived from a different molecule, or all three domains are derived from different molecules. In other words, the immunosuppressive molecule of the present invention is not a naturally occurring full-length ligand or receptor itself.

The term "ligand or receptor" refers to any molecule or atom capable of binding to an immunosuppressive protein and interacting with it. A ligand or receptor can be a naturally occurring molecule such as an organic or inorganic molecule, or a synthetic molecule. For example, the known ligands of NKG2A include HLA-E, the known ligands of TIM3 include Galectin9, HMGB1 and CEACAM1, the known ligands of LAG3 include Galectin3, LSECtin, FGL1 and certain MHC class II molecules, the known ligands of TIGIT include CD112, CD113, CD155 and Nectin4, the known ligands of CTLA4 include CD80 and CD86, the known ligands of PD1 include PDL1 and PDL2, the known ligands of SIRPα include CD47, and the known ligands of LILRB1 include HLA-G.

Unless the context clearly dictates otherwise, the "binding domain" of the present invention encompasses the antibodies, ligands and functional fragments thereof as described above. Therefore, the binding domain as described in the present invention is selected from the group consisting of an intact antibody, Fab, Fab', F(ab')2, an Fd fragment, Fd', an Fv fragment, scFv, sdFv, a linear antibody, a diabody, sdAbs, and a functional fragment of a ligand or a receptor.

The term "functional variant" or "functional fragment" refers to a variant or fragment that contains at least one amino acid modification (i.e., substitution, deletion, or insertion) compared to the parent amino acid sequence but retains the biological activity of the parent amino acid sequence. For example, the functional fragment of a ligand or a receptor in the present invention generally refers to a fragment of a ligand or a receptor capable of binding to the corresponding PD1 or immunosuppressive protein, e.g. an extracellular region.

The term "heavy chain" refers to the larger of the two types of polypeptide chains present in a naturally occurring conformation in an antibody molecule and generally determines the class to which the antibody pertains. The term "light chain" refers to the smaller of the two types of polypeptide chains present in a naturally occurring conformation in an antibody molecule. Kappa (κ) and lambda (λ) light chains refer to the two major antibody light chain isotypes.

The term "complementarity determining region" or "CDR" refers to the amino acid sequence within the variable region of an antibody that confers antigen specificity and binding affinity. For example, generally, there are three CDRs present in each heavy chain variable region (e.g., CDR1-H, CDR2-H, and CDR3-H), and three CDRs in each light chain variable region (CDR1-L, CDR2-L, and CDR3-L). The precise amino acid sequence boundaries for a CDR can be determined using any of a number of well-known schemes, including: the Kabat numbering scheme, the Chothia numbering scheme, the IMGT numbering scheme, the AHo numbering scheme, and the AbM numbering scheme. The precise amino acid sequence for a given CDR or FR may vary depending on the numbering scheme chosen. It is understood that a "CDR" or "FR" of a given antibody or region thereof (e.g., a variable region thereof) encompasses CDRs or FRs as defined by any of the above or other known schemes. Where a designated CDR or FR contains a given amino acid sequence, it is understood that such CDR or FR may also have the sequence of the corresponding CDR or FR as defined by any of the above or other known schemes. The numbering scheme used herein to define the boundaries of CDRs and FRs is the Chothia scheme.

The term "single chain antibody" or "scFv" refers to a fusion protein comprising at least one light chain variable region (VL) and at least one heavy chain variable region (VH), wherein the light and heavy chain variable regions are contiguous (e.g., linked via a linker) and capable of being expressed as a single chain polypeptide, and wherein the scFv retains the specificity of the intact antibody from which it is derived. Unless otherwise specified, the scFv herein may have the VL and VH in any sequence (e.g., relative to the the N-terminus and C-terminus of the polypeptide), and the scFv may contain either VL-linker-VH or VH-linker-VL from the N-terminus to the C-terminus. The term "linker" refers to a molecular sequence that links two molecules or two sequences within the same molecule. In some embodiments, the linker is a peptide linker. Preferably, the linker does not adversely affect the expression, secretion, or biological activity of the polypeptide. In addition, the linker is preferably not antigenic and does not cause immune response. In some embodiments, the linker may be an endogenous amino acid sequence, an exogenous amino acid sequence (e.g., a GS-rich sequence), or a non-peptide chemical linker. In an embodiment, the linker of the present invention has at least 70%, preferably at least 80%, more preferably at least 90%, 95%, 97% or 99%, or 100% sequence identity to the amino acid sequence as set forth in SEQ ID NO: 111 or 112.

The term "single domain antibody or "sdAb" refers to a single antigen binding polypeptide with three complementarity determining regions (CDRs), which includes full-length antibodies (e.g., HCAbs) and antigen binding fragments thereof (e.g., VH, VL, VHH). In some cases, the single domain antibody is selected from or engineered based on camelid- or shark-derived HCAb, and its heavy chain variable domain is referred to herein as "VHH". VHH has the following basic structure from N-terminus to C-terminus: FR1-CDR1-FR2-CDR2-FR3-CDR3-FR4, wherein FR1 to FR4 refer to framework regions 1 to 4, respectively, and CDR1 to CDR3 refer to complementarity determining regions 1 to 3.

### PD1

In some embodiments, the PD1-binding molecule of the present invention comprises a PD1-binding domain, the PD1-binding domain being a PD1-targeting antibody, a PD1-binding ligand, or a functional fragment thereof. PD1, also known as CD279, is a member of the immunoglobulin superfamily and a type I transmembrane protein primarily expressed on activated T cells, NK cells, B cells, macrophages, dendritic cells, and monocytes, comprising an extracellular immunoglobulin variable domain, a transmembrane domain, and a cytoplasmic tail domain. The cytoplasmic tail domain thereof contains two tyrosine-based motifs: an immunoreceptor tyrosine-based inhibition motif (ITIM) and an immunoreceptor inhibitory tyrosine-based switch motif (ITSM). Studies have shown that the ITSM is necessary for PD1 to exert the immunosuppressive function on activated T cells. Ligands for PD1 include PDL1 (B7-H1, CD274) and PDL2 (B7-DC, CD273), both of which pertain to the protein B7 family. PD1 exerts negative immune regulatory effect through interaction with its ligands, PDL1 and PDL2. When PD1 interacts with its ligands, its intracellular ITSM becomes phosphorylated and recruits the corresponding phosphatases SHP-1 and SHP-2, leading to dephosphorylation of downstream signaling molecules and thus downregulating immune cell response levels. This negative regulatory mechanism of the immune system is a key molecular basis for maintaining immune tolerance. Numerous studies have shown that the immunosuppressive state of an organism induced by the overexpression of negative immune regulatory molecules such as PD1 and its interaction with the ligands PD-L1/PD-L2 plays an important role in the pathogenesis of cancer and chronic infectious diseases such as HIV, HCV, and HBV.

In some embodiments, the PD1 binding domain is a PD1-targeting antibody. Anti-PD1 antibodies known in the art can all be used in the present invention. In some embodiments, the PD1-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 75; and the CDR1-L, CDR2-L and CDR3-L comprised in the light chain variable region are the same as the CDR1-L, CDR2-L and CDR3-L comprised in SEQ ID NO: 76. In some embodiments, CDR1-H comprised in the heavy chain variable region is as set forth in SEQ ID NO: 69, CDR2-H is as set forth in SEQ ID NO: 70, and CDR3-H is as set forth in SEQ ID NO: 71; CDR1-L comprised in the light chain variable region is as set forth in SEQ ID NO: 72, CDR2-L is as set forth in SEQ ID NO: 73, and CDR3-L is as set forth in SEQ ID NO: 74.

In some embodiments, the PD1-targeting antibody comprises a light chain variable region and a heavy chain variable region, wherein the heavy chain variable region has at least 90%, at least 91%, at least 92%, at least 93%, at least 94%, at least 95%, at least 96%, at least 97%, at least 98%, at least 99%, or 100% identity to SEQ ID NO: 75; wherein the light chain variable region has at least 90%, at least 91%, at least 92%, at least 93%, at least 94%, at least 95%, at least 96%, at least 97%, at least 98%, at least 99%, or 100% identity to SEQ ID NO: 76. Preferably, the PD1-targeting antibody of the present invention comprises a heavy chain variable region as set forth in SEQ ID NO: 75 and a light chain variable region as set forth in SEQ ID NO:76.

In some embodiments, the PD1-targeting antibody has at least 90%, at least 91%, at least 92%, at least 93%, at least 94%, at least 95%, at least 96%, at least 97%, at least 98%, at least 99%, or 100% identity to SEQ ID NO: 77. Preferably, the PD1-targeting antibody is as set forth in SEQ ID NO: 77.

In some other embodiments, the PD1-binding domain comprised in the PD1-binding molecule of the present invention is a PD1-binding ligand, for example, PDL1, PDL2 or a functional fragment thereof (i.e., a fragment having PD1-binding function, for example the extracellular region of PDL1 or PDL2). Preferably, the PD1-binding ligand comprises an amino acid sequence having at least 90%, at least 91%, at least 92%, at least 93%, at least 94%, at least 95%, at least 96%, at least 97%, at least 98%, at least 99%, or 100% identity to SEQ ID NO: 78 or 79. More preferably, the PD1-binding ligand comprises the amino acid sequence as set forth in SEQ ID NO: 78 or 79. In this embodiment, the transmembrane region of the PD1-binding molecule is preferably a transmembrane region of PDL1 or PDL2, and optionally, the PD1-binding molecule comprises an intracellular region of PDL1 or PDL2.

### NKG2A

In some embodiments, the immunosuppressive molecule of the present invention comprises an NKG2A binding domain, the NKG2A binding domain being an NKG2A-targeting antibody, an NKG2A-binding ligand, or a functional fragment thereof. NKG2A, also known as KLRC1 or CD159A, is a member of the C-type lectin superfamily and a type II transmembrane protein primarily expressed in cytotoxic lymphocytes (e.g. CD8+ T cells and NK cells), comprising a cytoplasmic domain, a transmembrane domain, and an extracellular lectin-like domain. Its intracellular portion contains two immunoreceptor tyrosine-based inhibitory motifs (ITIMs), which are involved in inhibitory signal transduction. NKG2A is capable of forming a heterodimeric receptor with CD94, and NKG2A/CD94 transmits inhibitory signals to NK cells and CD8+ T cells, etc. The natural ligand for NKG2A is HLA-E, and the interaction between NKG2A and HLA-E contributes to tumor immune evasion, and disrupting the interaction between NKG2A and HLA-E has been demonstrated to effectively enhance anti-tumor immune response. Additionally, more and more study results show that NKG2A also plays an important role in other immune-related diseases, including viral infections, autoimmune diseases, inflammatory diseases, parasitic infections, etc.

In some embodiments, the NKG2A binding domain is an NKG2A-targeting antibody. Anti-NKG2A antibodies known in the art can all be used in the present invention. In some embodiments, the NKG2A-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 7; and the CDR1-L, CDR2-L and CDR3-L comprised in the light chain variable region are the same as the CDR1-L, CDR2-L and CDR3-L comprised in SEQ ID NO: 8. In some embodiments, CDR1-H comprised in the heavy chain variable region is as set forth in SEQ ID NO: 1, CDR2-H is as set forth in SEQ ID NO: 2, and CDR3-H is as set forth in SEQ ID NO: 3; CDR1-L comprised in the light chain variable region is as set forth in SEQ ID NO: 4, CDR2-L is as set forth in SEQ ID NO: 5, and CDR3-L is as set forth in SEQ ID NO: 6.

In some embodiments, the NKG2A-targeting antibody comprises a light chain variable region and a heavy chain variable region, wherein the heavy chain variable region has at least 90%, at least 91%, at least 92%, at least 93%, at least 94%, at least 95%, at least 96%, at least 97%, at least 98%, at least 99%, or 100% identity to SEQ ID NO: 7; wherein the light chain variable region has at least 90%, at least 91%, at least 92%, at least 93%, at least 94%, at least 95%, at least 96%, at least 97%, at least 98%, at least 99%, or 100% identity to SEQ ID NO: 8. Preferably, the NKG2A-targeting antibody of the present invention comprises a heavy chain variable region as set forth in SEQ ID NO: 7 and a light chain variable region as set forth in SEQ ID NO:8.

In some embodiments, the NKG2A-targeting antibody has at least 90%, at least 91%, at least 92%, at least 93%, at least 94%, at least 95%, at least 96%, at least 97%, at least 98%, at least 99%, or 100% identity to SEQ ID NO: 9. Preferably, the NKG2A-targeting antibody is as set forth in SEQ ID NO: 9.

In some other embodiments, the NKG2A binding domain comprised in the immunosuppressive molecule of the present invention is an NKG2A-binding ligand, for example, HLA-E or a functional fragment thereof (i.e., a fragment having NKG2A binding function, for example the extracellular region of HLA-E). Preferably, the NKG2A-binding ligand comprises an amino acid having at least 90%, at least 91%, at least 92%, at least 93%, at least 94%, at least 95%, at least 96%, at least 97%, at least 98%, at least 99%, or 100% identity to SEQ ID NO: 10 or 11. More preferably, the NKG2A-binding ligand comprises the amino acid sequence as set forth in SEQ ID NO: 10 or 11. In this embodiment, the transmembrane region of the immunosuppressive molecule may be the transmembrane region of HLA-E. However, typically, the immunosuppressive molecule of this embodiment does not comprise the intracellular region of HLA-E. In other words, the immunosuppressive molecule is not full-length HLA-E.

### TIM3

In some embodiments, the immunosuppressive molecule of the present invention comprises a TIM3 binding domain, the TIM3 binding domain being a TIM3-targeting antibody, a TIM3-binding ligand, or a functional fragment thereof. TIM3, also known as HAVCR2 or CD366, is a member of the TIM family and a type I transmembrane protein expressed in a variety of immune cells (e.g. Th1 cells, Th17 cells, monocytes, dendritic cells, macrophages, etc.), comprising an extracellular immunoglobulin variable domain, a mucin domain, a transmembrane domain, and an intracellular domain. Studies have found that TIM3 binds to different ligands (e.g. Galectin9, HMGB1, CEACAM1), triggering signal transduction in immune cells. Galectin9 has high affinity for the TIM3 IgV domain, and the TIM3/Galectin9 signaling pathway is capable of mediating T cell exhaustion. In addition, Galectin9 can promote the maturation of TIM3+ antigen-presenting cells, thereby enhancing adaptive immunity. Therefore, the TIM3/Galectin9 signaling pathway plays an important role in organism immunomodulation. HMGB1 is a DNA binding protein that can function as a chromatin factor, and also plays an important role in inflammation, immune responses, DC differentiation, maintenance of genomic stability, and nuclear transcriptional regulation. HMGB1 acts as a ligand of TIM3, in the tumor microenvironment, tumor-infiltrating DCs highly expressing TIM3 protein competitively bind to HMGB1 against nucleic acids released by tumor cells, preventing nucleic acids from entering endosomes, thereby suppressing nucleic acid-stimulated innate immune response. Therefore, the activation and function of innate immune cells are disrupted, and blocking TIM3-mediated nucleic acid signaling may contribute to DNA vaccine research. CEACAM1, as a ligand for TIM3, binds to TIM3 through its N-terminal domain to form a cis-heterodimer, promoting stable expression of TIM3 on the surface of immune cells. However, trans-interaction between CEACAM1 and TIM3 is capable of suppressing the function of immune cells. Both cis- and trans-interactions between CEACAM1 and TIM3 could maintain the tolerance of immune cells. Study results have shown that combined blockade of the CEACAM1 and TIM3 pathways can enhance anti-tumor immune responses and improve tumor clearance rates in a patient with colorectal cancer. Therefore, the interaction between CEACAM1 and TIM3 plays a key role in regulating autoimmunity and anti-tumor immunity.

In some embodiments, the TIM3 binding domain is a TIM3-targeting antibody. Anti-TIM3 antibodies known in the art can all be used in the present invention. In some embodiments, the TIM3-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 19; and the CDR1-L, CDR2-L and CDR3-L comprised in the light chain variable region are the same as the CDR1-L, CDR2-L and CDR3-L comprised in SEQ ID NO: 20. In some embodiments, CDR1-H comprised in the heavy chain variable region is as set forth in SEQ ID NO: 13, CDR2-H is as set forth in SEQ ID NO: 14, and CDR3-H is as set forth in SEQ ID NO: 15; CDR1-L comprised in the light chain variable region is as set forth in SEQ ID NO: 16, CDR2-L is as set forth in SEQ ID NO: 17, and CDR3-L is as set forth in SEQ ID NO: 18.

In some embodiments, the TIM3-targeting antibody comprises a light chain variable region and a heavy chain variable region, wherein the heavy chain variable region has at least 90%, at least 91%, at least 92%, at least 93%, at least 94%, at least 95%, at least 96%, at least 97%, at least 98%, at least 99%, or 100% identity to SEQ ID NO: 19; wherein the light chain variable region has at least 90%, at least 91%, at least 92%, at least 93%, at least 94%, at least 95%, at least 96%, at least 97%, at least 98%, at least 99%, or 100% identity to SEQ ID NO: 20. Preferably, the TIM3-targeting antibody of the present invention comprises a heavy chain variable region as set forth in SEQ ID NO: 19 and a light chain variable region as set forth in SEQ ID NO:20.

In some embodiments, the TIM3-targeting antibody has at least 90%, at least 91%, at least 92%, at least 93%, at least 94%, at least 95%, at least 96%, at least 97%, at least 98%, at least 99%, or 100% identity to SEQ ID NO: 21. Preferably, the TIM3-targeting antibody is as set forth in SEQ ID NO: 21.

In some other embodiments, the TIM3 binding domain comprised in the immunosuppressive molecule of the present invention is a TIM3-binding ligand, for example, Galectin9, HMGB1, CEACAM1 or a functional fragment thereof (i.e., a fragment having TIM3 binding function, for example the extracellular region of CEACAM1). Preferably, the TIM3-binding ligand comprises an amino acid sequence having at least 90%, at least 91%, at least 92%, at least 93%, at least 94%, at least 95%, at least 96%, at least 97%, at least 98%, at least 99%, or 100% identity to the amino acid sequence as set forth in any one of SEQ ID NOs: 22-24. More preferably, the TIM3-binding ligand comprises the amino acid sequence as set forth in any one of SEQ ID NOs: 22-24. In a specific embodiment, the TIM3-binding ligand comprises an extracellular region of CEACAM1 as set forth in SEQ ID NO: 24. In this embodiment, the transmembrane region of the immunosuppressive molecule is preferably the transmembrane region of CEACAM1. However, typically, the immunosuppressive molecule of this embodiment does not comprise the intracellular region of CEACAM1. In other words, the immunosuppressive molecule is not full-length CEACAM1.

### LAG3

In some embodiments, the immunosuppressive molecule of the present invention comprises an LAG3 binding domain, the LAG3 binding domain being an LAG3-targeting antibody, an LAG3-binding ligand, or a functional fragment thereof. LAG3, also known as CD223, is a type I transmembrane protein, pertaining to the immunoglobulin superfamily (IgSF). It is expressed as dimers or oligomers on the surfaces of activated CD4+ and CD8+ T cells, regulatory T cells, Tr1 cells, NK cells, and plasmacytoid dendritic cells (pDCs). The LAG3 protein shares a homologous structure with CD4, with both having four Ig-like domains in the extracellular regions. However, LAG3 contains an additional looped structure within the membrane-distal Ig-like domain, which is absent in CD4. This extra looped structure enables LAG3 protein to bind to MHC class II molecules (e.g. HLA-DR, HLA-DP, and HLA-DQ antigens, etc.) expressed on antigen-presenting cells (APCs) or tumor cells, which has significantly higher affinity than CD4, and can negatively regulate T cell receptor (TCR) signaling. In addition to MHC class II molecules, Galectin-3, LSECtin, and FGL1 are also LAG3 ligands. These three proteins are expressed on various tumor cells and suppress T cell response. Studies have shown that LAG3 can negatively regulate T cell activation, proliferation, and cytokine production, suppress pDC activation, and enhance the suppressive activity of regulatory T cells.

In some embodiments, the LAG3 binding domain is a LAG3-targeting antibody. Anti-LAG3 antibodies known in the art can all be used in the present invention. In some embodiments, the LAG3-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 32; and the CDR1-L, CDR2-L and CDR3-L comprised in the light chain variable region are the same as the CDR1-L, CDR2-L and CDR3-L comprised in SEQ ID NO: 33. In some embodiments, CDR1-H comprised in the heavy chain variable region is as set forth in SEQ ID NO: 26, CDR2-H is as set forth in SEQ ID NO: 27, and CDR3-H is as set forth in SEQ ID NO: 28; CDR1-L comprised in the light chain variable region is as set forth in SEQ ID NO: 29, CDR2-L is as set forth in SEQ ID NO: 30, and CDR3-L is as set forth in SEQ ID NO: 31.

In some embodiments, the LAG3-targeting antibody comprises a light chain variable region and a heavy chain variable region, wherein the heavy chain variable region has at least 90%, at least 91%, at least 92%, at least 93%, at least 94%, at least 95%, at least 96%, at least 97%, at least 98%, at least 99%, or 100% identity to SEQ ID NO: 32; wherein the light chain variable region has at least 90%, at least 91%, at least 92%, at least 93%, at least 94%, at least 95%, at least 96%, at least 97%, at least 98%, at least 99%, or 100% identity to SEQ ID NO: 33. Preferably, the LAG3-targeting antibody of the present invention comprises a heavy chain variable region as set forth in SEQ ID NO: 32 and a light chain variable region as set forth in SEQ ID NO:33.

In some embodiments, the LAG3-targeting antibody has at least 90%, at least 91%, at least 92%, at least 93%, at least 94%, at least 95%, at least 96%, at least 97%, at least 98%, at least 99%, or 100% identity to SEQ ID NO: 34. Preferably, the LAG3-targeting antibody is as set forth in SEQ ID NO: 34.

In some other embodiments, the LAG3 binding domain comprised in the immunosuppressive molecule of the present invention is an LAG3-binding ligand, for example, an MHC class II molecule, Galectin 3, LSECtin, FGL1, or a functional fragment thereof (i.e., a fragment having LAG3 binding function, for example the extracellular region of LSECtin). Preferably, the LAG3-binding ligand comprises an amino acid sequence having at least 90%, at least 91%, at least 92%, at least 93%, at least 94%, at least 95%, at least 96%, at least 97%, at least 98%, at least 99%, or 100% identity to the amino acid sequence as set forth in any one of SEQ ID NOs: 35-37. More preferably, the LAG3-binding ligand comprises the amino acid sequence as set forth in any one of SEQ ID NOs: 35-37. In a specific embodiment, the LAG3-binding ligand comprises an extracellular region of LSECtin as set forth in SEQ ID NO: 37. In this embodiment, the transmembrane region of the immunosuppressive molecule is preferably the transmembrane region of LSECtin. However, typically, the immunosuppressive molecule of this embodiment does not comprise the intracellular region of LSECtin. In other words, the immunosuppressive molecule is not full-length LSECtin.

### TIGIT

In some embodiments, the immunosuppressive molecule of the present invention comprises a TIGIT binding domain, the TIGIT binding domain being a TIGIT-targeting antibody, a TIGIT-binding ligand, or a functional fragment thereof. TIGIT, also known as VSTM3, VSIG9, or WUCAM, is a member of the PVR-like protein family, and a type I transmembrane protein primarily expressed in NK cells and T cells (e.g. CD4+ T cells, CD8+ T cells, and Treg cells, etc.), comprising an extracellular immunoglobulin variable domain, a transmembrane domain, and a short intracellular domain, which having an immunoreceptor tyrosine-based inhibitory motif (ITIM) and an immunoglobulin tyrosine-based tail (ITT)-like motif. TIGIT has four ligands: CD112, CD113, CD155, and Nectin4, which all pertain to the Nectin/NECL family, the family participating in mediating cell adhesion, cell polarization, and other processes. TIGIT binds to CD155 and Nectin4 with significantly higher affinity than CD112 and CD113. At the same time, CD155 also functions as a ligand for the activating receptor CD226. When CD155 binds to CD226, it transmits an activating signal to immune cells. When CD155 binds to TIGIT, it recruits the SHP1 phosphatase to the membrane through its ITIM motif to transmit a inhibitory signal, subsequently inactivating numerous proteins involved in T cell effector functions. Nectin4 is the only member of the Nectin/NECL family of proteins that could specifically bind to TIGIT without interacting with CD226, CD96, or CD112R.

In some embodiments, the TIGIT binding domain is a TIGIT-targeting antibody. Anti-TIGIT antibodies known in the art can all be used in the present invention. In some embodiments, the TIGIT-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 45; and the CDR1-L, CDR2-L and CDR3-L comprised in the light chain variable region are the same as the CDR1-L, CDR2-L and CDR3-L comprised in SEQ ID NO: 46. In some embodiments, CDR1-L comprised in the light chain variable region is as set forth in SEQ ID NO: 39, CDR2-L is as set forth in SEQ ID NO: 40, and CDR3-L is as set forth in SEQ ID NO: 41; CDR1-H comprised in the heavy chain variable region is as set forth in SEQ ID NO: 42, CDR2-H is as set forth in SEQ ID NO: 43, and CDR3-H is as set forth in SEQ ID NO: 44.

In some embodiments, the TIGIT-targeting antibody comprises a light chain variable region and a heavy chain variable region, wherein the heavy chain variable region has at least 90%, at least 91%, at least 92%, at least 93%, at least 94%, at least 95%, at least 96%, at least 97%, at least 98%, at least 99%, or 100% identity to SEQ ID NO: 45; wherein the light chain variable region has at least 90%, at least 91%, at least 92%, at least 93%, at least 94%, at least 95%, at least 96%, at least 97%, at least 98%, at least 99%, or 100% identity to SEQ ID NO: 46. Preferably, the TIGIT-targeting antibody of the present invention comprises a heavy chain variable region as set forth in SEQ ID NO: 45 and a light chain variable region as set forth in SEQ ID NO:46.

In some embodiments, the TIGIT-targeting antibody has at least 90%, at least 91%, at least 92%, at least 93%, at least 94%, at least 95%, at least 96%, at least 97%, at least 98%, at least 99%, or 100% identity to SEQ ID NO: 47. Preferably, the TIGIT-targeting antibody is as set forth in SEQ ID NO: 47.

In some other embodiments, the TIGIT binding domain comprised in the immunosuppressive molecule of the present invention is a TIGIT-binding ligand, for example, CD155, CD112, CD113, Nectin4, or a functional fragment thereof (i.e., a fragment having TIGIT binding function, for example the extracellular region of CD155, CD112, CD113, or Nectin4). Preferably, the TIGIT-binding ligand comprises an amino acid sequence having at least 90%, at least 91%, at least 92%, at least 93%, at least 94%, at least 95%, at least 96%, at least 97%, at least 98%, at least 99%, or 100% identity to the amino acid sequence as set forth in any one of SEQ ID NOs: 48-51. More preferably, the TIGIT-binding ligand comprises the amino acid sequence as set forth in any one of SEQ ID NOs: 48-51. In this embodiment, the transmembrane region of the immunosuppressive molecule can be the corresponding transmembrane region of CD155, CD112, CD113, or Nectin4. However, typically, the immunosuppressive molecule of this embodiment does not comprise the corresponding intracellular region of CD155, CD112, CD113, or Nectin 4. In other words, the immunosuppressive molecule is not full-length CD155, CD112, CD113 or Nectin 4.

### CTLA4

In some embodiments, the immunosuppressive molecule of the present invention comprises a CTLA4 binding domain, the CTLA4 binding domain being a CTLA4-targeting antibody, a CTLA4-binding ligand, or a functional fragment thereof. CTLA4, also known as CD152 or CELIAC3, is a member of the CD28 immunoglobulin subfamily and a type I transmembrane protein primarily expressed on T cells, comprising an extracellular immunoglobulin variable domain, a transmembrane domain, and a conserved cytoplasmic tail region. The extracellular domain of the CD28 immunoglobulin subfamily contains a highly conserved motif (MYPPPY), which is crucial for binding its ligands CD80 and CD86. Ligands for CTLA4, CD80 and CD86, are typically found on the surface of antigen-presenting cells and can bind CD28 or CTLA4, respectively, to generate costimulatory or cosuppressive responses, but CD80 and CD86 bind CTLA4 with higher affinity. Due to this suppressive effect, CTLA4 is a key regulator for regulating T cell homeostasis and selftolerance.

In some embodiments, the CTLA4 binding domain is a CTLA4-targeting antibody. Anti-CTLA4 antibodies known in the art can all be used in the present invention. In some embodiments, the CTLA4-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 62; and the CDR1-L, CDR2-L and CDR3-L comprised in the light chain variable region are the same as the CDR1-L, CDR2-L and CDR3-L comprised in SEQ ID NO: 63. In some embodiments, CDR1-H comprised in the heavy chain variable region is as set forth in SEQ ID NO: 56, CDR2-H is as set forth in SEQ ID NO: 57, and CDR3-H is as set forth in SEQ ID NO: 58; CDR1-L comprised in the light chain variable region is as set forth in SEQ ID NO: 59, CDR2-L is as set forth in SEQ ID NO: 60, and CDR3-L is as set forth in SEQ ID NO: 61.

In some embodiments, the CTLA4-targeting antibody comprises a light chain variable region and a heavy chain variable region, wherein the heavy chain variable region has at least 90%, at least 91%, at least 92%, at least 93%, at least 94%, at least 95%, at least 96%, at least 97%, at least 98%, at least 99%, or 100% identity to SEQ ID NO: 62; wherein the light chain variable region has at least 90%, at least 91%, at least 92%, at least 93%, at least 94%, at least 95%, at least 96%, at least 97%, at least 98%, at least 99%, or 100% identity to SEQ ID NO: 63. Preferably, the CTLA4-targeting antibody of the present invention comprises a heavy chain variable region as set forth in SEQ ID NO: 62 and a light chain variable region as set forth in SEQ ID NO:63.

In some embodiments, the CTLA4-targeting antibody has at least 90%, at least 91%, at least 92%, at least 93%, at least 94%, at least 95%, at least 96%, at least 97%, at least 98%, at least 99%, or 100% identity to SEQ ID NO: 64. Preferably, the CTLA4-targeting antibody is as set forth in SEQ ID NO: 64.

In some other embodiments, the CTLA4 binding domain comprised in the immunosuppressive molecule of the present invention is a CTLA4-binding ligand, for example, CD80, CD86 or a functional fragment thereof (i.e., a fragment having CTLA4 binding function, for example the extracellular region of CD80 or CD86). Preferably, the CTLA4-binding ligand comprises an amino acid sequence having at least 90%, at least 91%, at least 92%, at least 93%, at least 94%, at least 95%, at least 96%, at least 97%, at least 98%, at least 99%, or 100% identity to SEQ ID NO: 65 or 66. More preferably, the CTLA4-binding ligand comprises the amino acid sequence as set forth in SEQ ID NO: 65 or 66. In this embodiment, the transmembrane region of the immunosuppressive molecule is preferably the transmembrane region of CD80 or CD86. However, typically, the immunosuppressive molecule of this embodiment does not comprise the intracellular region of CD80 or CD86. In other words, the immunosuppressive molecule is not full-length CD80 or CD86.

### IRP60

In some embodiments, the immunosuppressive molecule of the present invention comprises a IRP60 binding domain, the IRP60 binding domain being a IRP60-targeting antibody, a IRP60-binding ligand, or a functional fragment thereof. IRP60, also known as CD300a, is a type I transmembrane inhibitory receptor. IRP60 is widely expressed on the surface of myeloid cells (for example, dendritic cells, mast cells, granulocytes, monocytes) and lymphocytes (T cells, B cells, etc.), and its expression level varies depending on the cell type and the degree of differentiation. IRP60 comprises four ITIMs, and the initiation of its inhibitory signal depends on the phosphorylation of tyrosine residues in the ITIMs. By interacting with a ligand aminophospholipid (for example, PtdEth and/or PtdSer), IRP60 is involved in the regulation of a variety of cell activities, for example, cell growth, proliferation, apoptosis, differentiation, immune response, etc. It is reported that IRP60 is closely related to the development of diseases selected from the group consisting of hematological malignancies (for example, leukemia, lymphoma, etc.), infectious diseases, allergic reactivity, chronic inflammatory response, etc.

In some embodiments, the IRP60-binding domain is a IRP60-targeting antibody, for example, TX41, TX49, etc. Anti-IRP60 antibodies known in the art can all be used in the present invention. In some embodiments, the IRP60-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 150; and the CDR1-L, CDR2-L and CDR3-L comprised in the light chain variable region are the same as the CDR1-L, CDR2-L and CDR3-L comprised in SEQ ID NO: 151. In some embodiments, CDR1-H comprised in the heavy chain variable region is as set forth in SEQ ID NO: 144, CDR2-H is as set forth in SEQ ID NO: 145, and CDR3-H is as set forth in SEQ ID NO: 146; CDR1-L comprised in the light chain variable region is as set forth in SEQ ID NO: 147, CDR2-L is as set forth in SEQ ID NO: 148, and CDR3-L is as set forth in SEQ ID NO: 149.

In some embodiments, the IRP60-targeting antibody comprises a light chain variable region and a heavy chain variable region, wherein the heavy chain variable region has at least 90%, at least 91%, at least 92%, at least 93%, at least 94%, at least 95%, at least 96%, at least 97%, at least 98%, at least 99%, or 100% identity to SEQ ID NO: 150; wherein the light chain variable region has at least 90%, at least 91%, at least 92%, at least 93%, at least 94%, at least 95%, at least 96%, at least 97%, at least 98%, at least 99%, or 100% identity to SEQ ID NO: 151. Preferably, the IRP60-targeting antibody of the present invention comprises a heavy chain variable region as set forth in SEQ ID NO: 150 and a light chain variable region as set forth in SEQ ID NO:151.

In some embodiments, the IRP60-targeting antibody has at least 90%, at least 91%, at least 92%, at least 93%, at least 94%, at least 95%, at least 96%, at least 97%, at least 98%, at least 99%, or 100% identity to SEQ ID NO: 152. Preferably, the IRP60-targeting antibody is as set forth in SEQ ID NO: 152.

### SIRPα

In some embodiments, the immunosuppressive molecule of the present invention comprises an SIRPα binding domain, the SIRPα binding domain being an SIRPα-targeting antibody, an SIRPα-binding ligand, or a functional fragment thereof. SIRPα, also known as signal regulatory protein a or CD172a, which is a transmembrane protein expressed on the surface of macrophages, dendritic cells, NK cells, and nerve cells, and its intracellular region contains an immunoreceptor tyrosine-based inhibitory motif (ITIM). The ligand of SIRPα is CD47, a cell surface protein that transmits a "don't eat me" signal on healthy cells. In addition, CD47 is also widely expressed on various tumor cells as a mechanism to evade immune detection. Many studies have found that inhibition of the CD47-SIRPα signaling pathway in cancer cells (for example, by anti-CD47 antibodies or anti-SIRPα antibodies) can promote phagocytosis of tumor cells by macrophages, ultimately limiting tumor growth.

In some embodiments, the SIRPα binding domain is an SIRPα-targeting antibody. Anti-SIRPα antibodies known in the art can all be used in the present invention. In some embodiments, the SIRPα-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 159; and the CDR1-L, CDR2-L and CDR3-L comprised in the light chain variable region are the same as the CDR1-L, CDR2-L and CDR3-L comprised in SEQ ID NO: 160. In some embodiments, CDR1-H comprised in the heavy chain variable region is as set forth in SEQ ID NO: 153, CDR2-H is as set forth in SEQ ID NO: 154, and CDR3-H is as set forth in SEQ ID NO: 155; CDR1-L comprised in the light chain variable region is as set forth in SEQ ID NO: 156, CDR2-L is as set forth in SEQ ID NO: 157, and CDR3-L is as set forth in SEQ ID NO: 158.

In some embodiments, the SIRPα-targeting antibody comprises a light chain variable region and a heavy chain variable region, wherein the heavy chain variable region has at least 90%, at least 91%, at least 92%, at least 93%, at least 94%, at least 95%, at least 96%, at least 97%, at least 98%, at least 99%, or 100% identity to SEQ ID NO: 159; wherein the light chain variable region has at least 90%, at least 91%, at least 92%, at least 93%, at least 94%, at least 95%, at least 96%, at least 97%, at least 98%, at least 99%, or 100% identity to SEQ ID NO: 160. Preferably, the SIRPα-targeting antibody of the present invention comprises a heavy chain variable region as set forth in SEQ ID NO: 159 and a light chain variable region as set forth in SEQ ID NO:160.

In some embodiments, the SIRPα-targeting antibody has at least 90%, at least 91%, at least 92%, at least 93%, at least 94%, at least 95%, at least 96%, at least 97%, at least 98%, at least 99%, or 100% identity to SEQ ID NO: 161. Preferably, the SIRPα-targeting antibody is as set forth in SEQ ID NO: 161.

In some other embodiment, the SIRPα binding domain comprised in the immunosuppressive molecule of the present invention is an SIRPα-binding ligand, for example, CD47 or a functional fragment thereof (i.e., a fragment having SIRPα-binding function, for example the extracellular region of CD47). Preferably, the SIRPα-binding ligand comprises an amino acid sequence having at least 90%, at least 91%, at least 92%, at least 93%, at least 94%, at least 95%, at least 96%, at least 97%, at least 98%, at least 99%, or 100% identity to SEQ ID NO: 163. More preferably, the SIRPα-binding ligand comprises the amino acid sequence as set forth in SEQ ID NO: 163. In this embodiment, the transmembrane region of the immunosuppressive molecule is preferably the transmembrane region of CD47. However, typically, the immunosuppressive molecule of this embodiment does not comprise the intracellular region of CD47. In other words, the immunosuppressive molecule is not full-length CD47.

### KIR2DL1/2/3

In some embodiments, the immunosuppressive molecule of the present invention comprises a KIR2DL1/2/3 binding domain, the KIR2DL1/2/3 binding domain being a KIR2DL1-targeting antibody, KIR2DL2-targeting antibody, or KIR2DL3-targeting antibody (i.e., KIR2DL1/2/3-targeting antibody), a KIR2DL1/2/3-binding receptor, or a functional fragment thereof. Based on the number of Ig-like domains comprised in the extracellular region, KIR may be classified into KIR2D and KIR3D subfamilies. Based on the length of the cytoplasmic region, KIR may also be classified into long (L) type and short (S) type, for example, KIR2DL, KIR2DS, KIR3DL, and KIR3DS. Among these, the cytoplasmic regions of KIR2DL1, KIR2DL2, and KIR2DL3 comprise two immunoreceptor tyrosine-based inhibitory motifs (ITIMs), pertaining to inhibitory KIR receptors. Specifically, when ITIM comprised in the cytoplasmic regions of KIR2DL1, KIR2DL2, and KIR2DL3 is phosphorylated, it may recruit phosphatases SHP1 and SHP2, leading to dephosphorylation of cellular substrates and ultimately suppressing or terminating the effector functions of NK cells, for example, cytotoxicity and cytokine secretion.

In some embodiments, the KIR2DL1/2/3 binding domain is a KIR2DL1/2/3-targeting antibody. Anti-KIR2DL1/2/3 antibodies known in the art can all be used in the present invention. In some embodiments, the KIR2DL1/2/3-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 119 or 122; and the CDR1-L, CDR2-L and CDR3-L comprised in the light chain variable region are the same as the CDR1-L, CDR2-L and CDR3-L comprised in SEQ ID NO: 120 or 123. In some embodiments, CDR1-H comprised in the heavy chain variable region is as set forth in SEQ ID NO: 113, CDR2-H is as set forth in SEQ ID NO: 114, and CDR3-H is as set forth in SEQ ID NO: 115; CDR1-L comprised in the light chain variable region is as set forth in SEQ ID NO: 116, CDR2-L is as set forth in SEQ ID NO: 117, and CDR3-L is as set forth in SEQ ID NO: 118.

In some embodiments, the KIR2DL1/2/3-targeting antibody comprises a light chain variable region and a heavy chain variable region, wherein the heavy chain variable region has at least 90%, at least 91%, at least 92%, at least 93%, at least 94%, at least 95%, at least 96%, at least 97%, at least 98%, at least 99%, or 100% identity to SEQ ID NO: 119 or 122; wherein the light chain variable region has at least 90%, at least 91%, at least 92%, at least 93%, at least 94%, at least 95%, at least 96%, at least 97%, at least 98%, at least 99%, or 100% identity to SEQ ID NO: 120 or 123. Preferably, the KIR2DL1/2/3-targeting antibody of the present invention comprises a heavy chain variable region as set forth in SEQ ID NO:119 and a light chain variable region as set forth in SEQ ID NO:120, or a heavy chain variable region as set forth in SEQ ID NO:122 and a light chain variable region as set forth in SEQ ID NO:123.

In some embodiments, the KIR2DL1/2/3-targeting antibody has at least 90%, at least 91%, at least 92%, at least 93%, at least 94%, at least 95%, at least 96%, at least 97%, at least 98%, at least 99%, or 100% identity to SEQ ID NO: 121 or 124. Preferably, the KIR2DL1/2/3-targeting antibody is as set forth in SEQ ID NO: 121 or 124.

### LILRB1

In some embodiments, the immunosuppressive molecule of the present invention comprises an LILRB1 binding domain, the LILRB1 binding domain being an LILRB1-targeting antibody, an LILRB1-binding receptor, or a functional fragment thereof. LILRB1, also known as ILT2 or CD85j, is an immunoglobulin-like receptor pertaining to the LILR family. LILRB1 is normally expressed on the surface of immune cells, particularly on monocytes, dendritic cells, T cells, and B cells. Ligands of LILRB1 include molecules such as HLA-G. HLA-G is a type of MHC class I molecule expressed on the surface of placental tissue, fetus, and certain tumor cells. LILRB1, through binding to its ligands, participates in regulating the activation state of immune cells and may play a role in immunomodulation, tolerance, and anti-inflammatory processes. This interaction is crucial for regulating immune responses and maintaining immune system homeostasis.

In some embodiments, the LILRB1 binding domain is an LILRB1-targeting antibody. Anti-LILRB1 antibodies known in the art can all be used in the present invention. In some embodiments, the LILRB1-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 131 or 140; and the CDR1-L, CDR2-L and CDR3-L comprised in the light chain variable region are the same as the CDR1-L, CDR2-L and CDR3-L comprised in SEQ ID NO: 132 or 141. In some embodiments, CDR1-H comprised in the heavy chain variable region is as set forth in SEQ ID NO:134, CDR2-H is as set forth in SEQ ID NO:135, and CDR3-H is as set forth in SEQ ID NO:136; CDR1-L comprised in the light chain variable region is as set forth in SEQ ID NO: 137, CDR2-L is as set forth in SEQ ID NO: 138, and CDR3-L is as set forth in SEQ ID NO: 139; or CDR1-H comprised in the heavy chain variable region is as set forth in SEQ ID NO: 125, CDR2-H is as set forth in SEQ ID NO: 126, and CDR3-H is as set forth in SEQ ID NO: 127; CDR1-L comprised in the light chain variable region is as set forth in SEQ ID NO: 128, CDR2-L is as set forth in SEQ ID NO: 129, and CDR3-L is as set forth in SEQ ID NO: 130.

In some embodiments, the LILRB1-targeting antibody comprises a light chain variable region and a heavy chain variable region, wherein the heavy chain variable region has at least 90%, at least 91%, at least 92%, at least 93%, at least 94%, at least 95%, at least 96%, at least 97%, at least 98%, at least 99%, or 100% identity to SEQ ID NO: 131 or 140; wherein the light chain variable region has at least 90%, at least 91%, at least 92%, at least 93%, at least 94%, at least 95%, at least 96%, at least 97%, at least 98%, at least 99%, or 100% identity to SEQ ID NO: 132 or 141. Preferably, the LILRB1-targeting antibody of the present invention comprises a heavy chain variable region as set forth in SEQ ID NO:131 and a light chain variable region as set forth in SEQ ID NO:132, or a heavy chain variable region as set forth in SEQ ID NO:140 and a light chain variable region as set forth in SEQ ID NO:141.

In some embodiments, the LILRB1-targeting antibody has at least 90%, at least 91%, at least 92%, at least 93%, at least 94%, at least 95%, at least 96%, at least 97%, at least 98%, at least 99%, or 100% identity to SEQ ID NO: 133 or 142. Preferably, the LILRB1-targeting antibody is as set forth in SEQ ID NO: 133 or 142.

The term sequence "identity" means the degree to which two (nucleotide or amino acid) sequences in alignment have the same residue at the same position, and is usually expressed as a percentage. Preferably, identity is determined over the entire length of the sequences being compared. Therefore, two copies of the exact same sequence have 100% identity. Those skilled in the art know that several algorithms can be used to determine sequence identity, for example, Blast (Altschul et al. (1997) Nucleic Acids Res. 25:3389-3402), Blast2 (Altschul et al. (1990) J. Mol. Biol. 215: 403-410), Smith-Waterman (Smith et al. (1981) J. Mol. Biol. 147:195-197) and Clustal W.

The term "transmembrane domain" refers to a polypeptide structure that enables the expression of a protein molecule on the cell surface, and anchors the target binding domain to the cell membrane. The transmembrane domain may be natural or synthetic, and also may be derived from any membrane-bound protein or transmembrane protein. The transmembrane domain is capable of signaling when a target binding domain binds to a target. The transmembrane domain particularly suitable for the present invention can be derived from, for example, a TCR α chain, a TCR β chain, a TCR γ chain, a TCR δ chain, a CD3 ζ subunit, a CD3 ε subunit, a CD3 γ subunit, a CD3 δ subunit, CD28, CD45, CD4, CD5, CD8α, CD9, CD16, CD22, CD33, CD37, CD47, CD64, CD80, CD86, CD94, CD112, CD113, CD134, CD137, CD154, CD155, KIRDS2, OX40, CD2, CD27, CD18, ICOS, 4-1BB, GITR, CD40, BAFFR, HVEM, SLAMF7, NKp80, CD160, BCMA, IL-2R β, IL-2R γ, IL-7R a, ITGA1, VLA1, CD49a, ITGA4, IA4, CD49D, ITGA6, VLA-6, CD49f, ITGAD, CDl ld, ITGAE, CD103, ITGAL, CDl la, ITGAM, CDl lb, ITGAX, CDl lc, ITGB1, CD29, ITGB2, CD18, LFA-1, ITGB7, TNFR2, DNAM1, SLAMF4, CD84, CD96, LSECtin, PDL1, PDL2, CEACAM1, MHC class I molecules, MHC class II molecules, CRT AM, Ly9, CD160, PSGL1, CDIOO, SLAMF6, SLAMF1, SLAMF8, CD162, LTBR, PAG/Cbp, NKp44, NKp30, NKp46, NKG2D or NKG2C. In some embodiments, the transmembrane domain is derived from the following molecules: CD8α, CD4, CD28 or 4-1BB, or the transmembrane domain may be synthetic and may mainly comprise hydrophobic residues such as leucine and valine. Preferably, the transmembrane domain is derived from CD28, which has at least 70%, preferably at least 80%, more preferably at least 90%, 95%, 97%, or 99%, or 100% sequence identity to the amino acid sequence as set forth in SEQ ID NO: 82, or the transmembrane domain is derived from CD8α, which has at least 70%, preferably at least 80%, more preferably at least 90%, 95%, 97%, or 99%, or 100% sequence identity to the amino acid sequence as set forth in SEQ ID NO: 83 or 84. In some other embodiments, the transmembrane domain comprised in the PD1-binding molecule or in the immunosuppressive molecule of the present invention is derived from a ligand or a receptor targeting a PD1-binding molecule or an immunosuppressive protein, for example HLA-E, CEACAM1, LSECtin, CD47, CD112, CD113, CD155, Nectin4, CD80, CD86, PDL1, PDL2 and some MHC class II molecules, etc. Preferably, the transmembrane domain has at least 70%, preferably at least 80%, more preferably at least 90%, 95%, 97%, or 99%, or 100% sequence identity to the amino acid sequence as set forth in any one of SEQ ID NOs: 12, 25, 38, 52, 53, 54, 55, 67, 68, 80, 81, and 162. More preferably, the transmembrane domain is linked to the extracellular domain of the corresponding ligand or receptor, preferably directly linked.

In some embodiments, a hinge region is further comprised between the binding domain and the transmembrane domain. The term "hinge region" generally refers to any oligopeptide or polypeptide that functions to link a transmembrane domain to an antibody. Specifically, the hinge region serves to provide greater flexibility and accessibility to the antibody. The hinge region may comprise up to 300 amino acids, preferably 10 to 100 amino acids and most preferably 25 to 50 amino acids. The hinge region may be completely or partially derived from a natural molecule, e.g., completely or partially from the extracellular region of CD8, CD4 or CD28, or completely or partially from an antibody constant region. Alternatively, the hinge region may be a synthetic sequence corresponding to a naturally occurring hinge sequence, or may be a completely synthetic hinge sequence. Preferably, the hinge region comprises a hinge region moiety of CD8α, CD28, an FcγRIIIα receptor, IgG4 or IgG1, more preferably a hinge from CD8α, CD28 or IgG4. In some embodiments, the hinge region is from CD28, which has at least 70%, preferably at least 80%, more preferably at least 90%, 95%, 97%, or 99%, or 100% sequence identity to the amino acid sequence as set forth in SEQ ID NO: 85. In some embodiments, the hinge region is from CD8α, which has at least 70%, preferably at least 80%, more preferably at least 90%, 95%, 97%, or 99%, or 100% sequence identity to the amino acid sequence as set forth in SEQ ID NO: 86 or 87. In some embodiments, the hinge region is from IgG4, which has at least 70%, preferably at least 80%, more preferably at least 90%, 95%, 97%, or 99%, or 100% sequence identity to the amino acid sequence as set forth in SEQ ID NO: 88.

The term "co-stimulatory domain" refers to at least a moiety of a protein that mediates intracellular signal transduction to induce immune response such as an effector function, which is an intracellular functional signaling domain from a co-stimulatory molecule, comprising the entire intracellular region of the co-stimulatory molecule, or a functional fragment thereof. A "co-stimulatory molecule" refers to a cognate binding partner that specifically binds to a co-stimulatory ligand, thereby mediating a co-stimulatory response (for example, proliferation and survival). The co-stimulatory signaling domain of any costimulatory molecule is suitable for the immunosuppressive molecule as described herein. The co-stimulatory molecule includes, but is not limited to, MHC class I molecules, BTLA, and Toll ligand receptors. The co-stimulatory domain of the present invention includes, but is not limited to, an intracellular region derived from the following proteins: LTB, CD94, TLR1, TLR2, TLR3, TLR4, TLR5, TLR6, TLR7, TLR8, TLR9, TLR10, CARD11, CD2, CD7, CD8, CD18, CD27, CD28, CD30, CD40, CD54, CD83, CD134, 4-1BB, CD270, CD272, B7-H3, ICOS, CD357, DAP10, DAP12, LAT, NKG2C, SLP76, PD1, LIGHT, TRIM, ZAP70 and a combination thereof. Preferably, the co-stimulatory domain of the CAR of the present invention is 4-1BB and/or CD28. In some embodiments, the co-stimulatory domain is from CD28, which has at least 70%, preferably at least 80%, more preferably at least 90%, 95%, 97%, or 99%, or 100% sequence identity to the amino acid sequence as set forth in SEQ ID NO: 89. In some embodiments, the co-stimulatory domain is from 4-1BB, which has at least 70%, preferably at least 80%, more preferably at least 90%, 95%, 97%, or 99%, or 100% sequence identity to the amino acid sequence as set forth in SEQ ID NO: 90 or 91.

The term "primary signaling domain" refers to a protein structure that acts together after antigen-receptor binding to trigger primary signaling, which is generally an intracellular sequence of a T cell receptor and a co-receptor. The primary signaling domain generally comprises one or more Immunoreceptor Tyrosine-based Activation Motifs (ITAM). The primary signaling domain of the present invention includes, but is not limited to, an intracellular region derived from the following proteins: FcRγ, FcRβ, CD3γ, CD3δ, CD3ε, CD3ζ, CD5, CD22, CD79a, CD79b, NFAM1, STAM1, STAM2, and CD66d. In some embodiments, the immunosuppressive molecule of the present invention does not comprise a CD3ζ intracellular region, for example, a CD3ζ intracellular region having at least 70%, preferably at least 80%, more preferably at least 90%, 95%, 97%, or 99%, or 100% sequence identity to the amino acid sequence as set forth in SEQ ID NO: 92 or 93.

In some embodiments, the PD1-binding molecule or the immunosuppressive molecule of the present invention further comprises a signal peptide such that when it is expressed in a cell, e.g. a T cell, the nascent protein is directed to the endoplasmic reticulum and subsequently to the cell surface. The core of the signal peptide may contain a long hydrophobic amino acid segment, which has a tendency to form a single α-helix. At the end of the signal peptide, there is usually an amino acid segment recognized and cleaved by a signal peptidase. The signal peptidase may cleave during or after translocation, so as to generate a free signal peptide and a mature protein. Then, the free signal peptide is digested by a specific protease. The signal peptides that may be used in the present invention is well known to those skilled in the art, for example, the signal peptide derived from B2M, CD8α, IgG1, GM-CSFRα, etc. In some embodiments, the signal peptide that can be used in the present invention is from B2M, which has at least 70%, preferably at least 80%, more preferably at least 90%, 95%, 97%, or 99%, or 100% sequence identity to the amino acid sequence as set forth in SEQ ID NO: 94. In some embodiments, the signal peptide that can be used in the present invention is from CD8α, which has at least 70%, preferably at least 80%, more preferably at least 90%, 95%, 97%, or 99%, or 100% sequence identity to the amino acid sequence as set forth in SEQ ID NO: 95 or 96.

In some embodiments, the engineered cell of the present invention further expresses a functional exogenous receptor (as defined below).

### Functional exogenous receptors

In some embodiments, the engineered cell expressing the immunosuppressive molecule of the present invention can further express a functional exogenous receptor. The functional exogenous receptor of the present invention comprises a chimeric antigen receptor (CAR), a T cell receptor (TCR), a T cell receptor fusion protein (TFP), a T cell antigen coupler (TAC) or an immune mobilization monoclonal T cell receptor (ImmTAC), etc., preferably a chimeric antigen receptor or a T cell receptor, more preferably a chimeric antigen receptor.

The term "T cell receptor" or "TCR" refers to a membrane protein complex that responds to antigen presentation and participates in T cell activation. The stimulation of TCR is triggered by the major histocompatibility complex molecule (MHC) on the antigen presenting cell, which presents antigen peptides to T cells and bind to the TCR complexs to induce a series of intracellular signaling. TCR is composed of six peptide chains that form heterodimers, which are generally divided into αβ type and γδ type. Each peptide chain includes a constant region and a variable region, wherein the variable region is responsible for binding to specific antigen and MHC molecules. The variable region of TCR may comprise an ligand binding domain or be operably linked to an ligand binding domain, wherein the definition of the ligand binding domain is as described below.

The term "chimeric antigen receptor" or "CAR" refers to an artificially constructed hybrid polypeptide that generally includes an antigen (e.g., a tumor antigen) binding domain (e.g., an antibody or antigen ligand), a transmembrane domain, an optional co-stimulatory domain, and a primary signaling domain, and each domain is linked by a linker. CARs are able to reposition the specificity and reactivity of T cells and other immune cells to a selected target in a non-MHC-restricted manner. In some embodiments, the functional exogenous receptor of the present invention is a chimeric antigen receptor, which comprises a tumor antigen binding domain, a transmembrane domain, and a primary signaling domain, optionally further comprises one or more co-stimulatory domains. In some embodiments, the chimeric antigen receptor further comprises one or more of the following structures: a signal peptide, a hinge region, a suicide gene, a switch structure, etc.

The term "T cell antigen coupler" or "TAC" includes three functional domains: (1) a tumor targeting domain, including a single chain antibody, a designed ankyrin repeat protein (DARPin), or other targeting groups; (2) an extracellular domain, a CD3-binding singlechain antibody, so that TAC receptors and TCR receptors are close to each other; and (3) a transmembrane domain and an intracellular domain of a CD4 co-receptor, wherein the intracellular domain is linked to protein kinase LCK, to catalyze the phosphorylation of the immunoreceptor tyrosine activation motif (ITAM) of the TCR complex as an initial step in T cell activation.

The term "T cell receptor fusion protein" or "TFP" refers to a recombinant polypeptide derived from components of TCR, usually composed of a TCR subunit and an antigen binding region linked thereto, and expressed on the cell surface. Wherein, the TCR subunit includes at least part of the TCR extracellular domain, the transmembrane domain, and the TCR intracellular signal domain.

The term "immune mobilizing monoclonal T cell receptor" or "ImmTAC" is composed of an engineered T cell receptor (TCR) and an anti-CD3 scFv, wherein: the engineered TCR can specifically recognize and bind to HLA-peptide complex on the surface of tumor cells with significantly improved affinity, and promotes T cell-mediated effector functions through the interaction of the scFv antibody fragment with CD3.

In some embodiments, the functional exogenous receptor comprises an extracellular domain that specifically recognizes an antigen (e.g., a tumor antigen). In some embodiments, the extracellular domain comprises an antibody that specifically binds an antigen or a ligand of the antigen. In some embodiments, the antigen is selected from the group consisting of: ALK, ADRB3, AKAP-4, APRIL, ASGPR1, BCMA, B7H3, B7H4, B7H6, bcr-abl, BORIS, BST2, BAFF-R, BTLA, CD2, CD3, CD4, CD5, CD7, CD8, CD19, CD20, CD22, CD24, CD25, CD28, CD30, CD33, CD38, CD40, CD44, CD44v6, CD44v7/8, CD47, CD52, CD56, CD57, CD58, CD70, CD72, CD79a, CD79b, CD80, CD81, CD86, CD97, CD123, CD133, CD137, CD 138, CD151, CD171, CD179a, CD300LF, CDH16, CSPG4, CS1, Claudin 6, Claudin18.1, Claudin 18.2, CEA, CEACAM6, CLL1, c-Met, CAIX, CXORF61, CA125, CYP1B1, CS1, ELF2M, EGFR, EPCAM, EGFRvIII, EphA2, ERG/TMPRSS2ETS fusion gene, ETV6-AML, EMR2, EGP2, EGP40, FAP, FAR, FBP, FLT3, FOSL1, FCRL5, FCAR, Flt3, Flt4, Frizzled, GD2, GD3, gp100, gp130, GM3, GPC2, GPC3, GPRC5D, GPR20, GloboH, GHRHR, GHR, GITR, Her2, HER3, HER-4, HMWMAA, HAVCR1, HPV E6,E7, HVEM, HIV-1Gag, HLA-A1, HLA-A2, IL6R, IL-11Ra, IL-13Ra, IGF-I receptor, LTPR, LIFRP, LRP5, IGLL1, IGF1R, KIT, Kappa Light Chain, KDR, LewisY, LMP2, LY6K, LAGE-1a, legumain, LCK, LAIR1, LILRA2, LY75, MSLN, MUC1, MUC16, MAGE-A1, MAGE3, MAD-CT-1, MelanA/MART1, ML-IAP, MYCN, mut hsp70-2, NCAM, NY-BR-1, NY-ESO-1, NA17, Notch-1-4, nAchR, NKG2D, NKG2D ligand, OY-TES1, OR51E2, OX40, PRSS21, PSCA, PD1, PD-L1, PD-L2, PSMA, Prostase, PAP, PDGFR-β, PCTA-1/galectin 8, p53, p53 mutant, prostein, PLAC1, PANX3, PAX3, PAX5, PTCH1, RANK, RAGE-1, ROR1, Ras mutant, RhoC, RU1, RU2, Robol, SSEA-4, SSX2, SART3, Sp17, TSHR, Tn Ag, TGS5, TEM1/CD248, TEM7R, TARP, TCRα, TCRβ, TGFBR1, TGFBR2, TNFRSF4, TWEAK-R, TLR7, TLR9, TAG72, TROP-2, Tie 2, TRP-2, TNFR1, TNFR2, TEM1, UPK2VEGFR, WT1, XAGE1, 5T4, 8H9, αvβ6 integrin, CA9, folate receptor α, ephrin B2, tyrosinase, fucosyl GM1, o-acetyl-GD2, folate receptor β, polysialic acid, sperm protein 17, survivin and telomerase, sarcoma translocation breakpoint, human telomerase reverse transcriptase/hTERT, androgen receptor, intestinal carboxylesterase, cyclin B1, fibronectin, tenascin, carcinoembryonic variant of tumor necrosis region, and any combination thereof. Preferably, the antigen is selected from CD7, CD19, CD20, CD22, CD30, CD33, CD38, CD123, CD138, CD171, MUC1, MSLN, AFP, folate receptor α, CEA, PSCA, PSMA, Her2, EGFR, IL-13Ra, GD2, NKG2D, Claudin 18.2, ROR1, EGFRvIII, CS1, BCMA and GPRC5D, more preferably selected from CD19, Claudin 18.2, MSLN, GPRC5D, ROR1, CD7 and BCMA.

In some embodiments, the functional exogenous receptor comprises an extracellular domain that specifically recognizes CD19, for example, a CD19-targeting antibody. CD19-targeting antibodies known in the art can all be used in the present invention. In some embodiments, the CD19-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 103; and the CDR1-L, CDR2-L and CDR3-L comprised in the light chain variable region are the same as the CDR1-L, CDR2-L and CDR3-L comprised in SEQ ID NO: 104. In some embodiments, CDR1-H comprised in the heavy chain variable region is as set forth in SEQ ID NO: 97, CDR2-H is as set forth in SEQ ID NO: 98, and CDR3-H is as set forth in SEQ ID NO: 99; CDR1-L comprised in the light chain variable region is as set forth in SEQ ID NO: 100, CDR2-L is as set forth in SEQ ID NO: 101, and CDR3-L is as set forth in SEQ ID NO: 102.

In some embodiments, the CD19-targeting antibody comprises a light chain variable region and a heavy chain variable region, wherein the heavy chain variable region has at least 90%, at least 91%, at least 92%, at least 93%, at least 94%, at least 95%, at least 96%, at least 97%, at least 98%, at least 99%, or 100% identity to SEQ ID NO: 103; wherein the light chain variable region has at least 90%, at least 91%, at least 92%, at least 93%, at least 94%, at least 95%, at least 96%, at least 97%, at least 98%, at least 99%, or 100% identity to SEQ ID NO: 104. Preferably, the CD19-targeting antibody of the present invention comprises a heavy chain variable region as set forth in SEQ ID NO: 103 and a light chain variable region as set forth in SEQ ID NO:104.

In some embodiments, the CD19-targeting antibody has at least 90%, at least 91%, at least 92%, at least 93%, at least 94%, at least 95%, at least 96%, at least 97%, at least 98%, at least 99%, or 100% identity to SEQ ID NO: 105. Preferably, the CD19-targeting antibody is as set forth in SEQ ID NO: 105.

In some embodiments, the functional exogenous receptor comprises an extracellular domain that specifically recognizes Claudin 18.2, for example, a Claudin 18.2-targeting antibody. Claudin 18.2-targeting antibodies known in the art can all be used in the present invention. In some embodiments, the Claudin 18.2-targeting antibody comprises a VHH, wherein the CDR1, CDR2 and CDR3 comprised in the VHH are the same as CDR1, CDR2 and CDR3 as comprised in SEQ ID NO: 109. In some embodiments, CDR1 comprised in the VHH is as set forth in SEQ ID NO: 106, CDR2 is as set forth in SEQ ID NO: 107, and CDR3 is as set forth in SEQ ID NO: 108.

In some embodiments, the Claudin 18.2-targeting antibody comprises an amino acid sequence having at least 90%, at least 91%, at least 92%, at least 93%, at least 94%, at least 95%, at least 96%, at least 97%, at least 98%, at least 99%, or 100% identity to SEQ ID NO: 109. Preferably, the Claudin 18.2-targeting antibody of the present invention comprises the amino acid sequence as set forth in SEQ ID NO: 109.

In some embodiments, the functional exogenous receptor of the present invention is a chimeric antigen receptor, which comprises an antigen binding domain, a transmembrane domain, and a primary signaling domain, optionally further comprises one or more costimulatory domains. In some embodiments, the functional exogenous receptor of the present invention is a chimeric antigen receptor, the chimeric antigen receptor targeting CD7, CD19, CD20, CD22, CD30, CD33, CD38, CD123, CD138, CD171, MUC1, MSLN, AFP, folate receptor α, CEA, PSCA, PSMA, Her2, EGFR, IL-13Ra, GD2, NKG2D, Claudin 18.2, ROR1, EGFRvIII, CS1, BCMA, GPRC5D, or any combination thereof, more preferably targeting CD19, Claudin 18.2, MSLN, ROR1, GPRC5D, CD7, BCMA, or any combination thereof. In some embodiments, the chimeric antigen receptor further comprises a signal peptide, a hinge region, or both. For the definitions of the transmembrane domain, costimulatory domain, primary signaling domain, and the optional hinge region, signal peptide and other structures that can be used in the CAR of the present invention, see the above "PD1-binding molecules and immunosuppressive molecules" section.

In some embodiments, the CAR of the present invention may further comprise a switch structure to regulate the expression time of the CAR. For example, the switch structure may be in the form of a dimerization domain, which undergoes a conformational change by binding to its corresponding ligand, exposing the extracellular binding domain, allowing it to bind to the targeted antigen, thereby activating the signal transduction pathway. Alternatively, switch domains may also be used to link the binding domain and the signaling domain, respectively, and only when the switch domains are coupled with each other (e.g., in the presence of an inducing compound), the binding domain and the signaling domain can be linked together through the dimerization, thereby activating the signaling pathway. The switch structure may further be in the form of a masking peptide. The masking peptide can mask the extracellular binding region and prevent it from binding to the targeted antigen. When the masking peptide is cleaved by, for example, protease, the extracellular binding region can be exposed, making it a "normal" CAR structure. Various switch structures known to those skilled in the art can all be used in the present invention.

In some embodiments, the CAR of the present invention may further comprise a suicide gene, i.e., to make it express a cell death signal that can be induced by an exogenous substance, so as to eliminate CAR cells when needed (e.g., when severe toxic side effects occur). For example, the suicide gene may be in the form of inserted epitopes, for example, CD20 epitopes, RQR8, etc., and when necessary, CAR cells can be eliminated by adding antibodies or reagents targeting these epitopes. The suicide gene may also be herpes simplex virus thymidine kinase (HSV-TK), which could cause cell death induced by ganciclovir treatment. The suicide gene may further be iCaspase-9, and the dimerization of iCaspase-9 can be induced by a chemically inducing medicament, e.g., AP1903, AP20187, etc., thereby activating the downstream Caspase3 molecule and leading to cell apoptosis. Various suicide genes known to those skilled in the art can all be used in the present invention.

### Cells

In some embodiments, the engineered cell of the present invention is an engineered immune cell.

The term "immune cell" refers to any cell of the immune system that has one or more effector functions (e.g., cytotoxic cell killing activity, secretion of cytokines, induction of ADCC and/or CDC). For example, the immune cell may be a B cell, a T cell, a macrophage, a dendritic cell, a monocyte, an NK cell, or an NKT cell. The immune cell may be obtained from a variety of sources, for example from a subject (e.g., isolated from peripheral blood monocytes, bone marrow, lymph node tissue, cord blood, thymus tissue, tissue from sites of infection, ascites, pleural effusion, spleen tissue, tumors, etc. of the subject), or from a cell line cultured in vitro (e.g., Jurkat, SupT1, NK92, etc.), or differentiated from stem cells (e.g., derived from cord blood stem cells, progenitor cells, bone marrow stem cells, hematopoietic stem cells, adult stem cells, embryonic stem cells, pluripotent stem cells, iPSCs, etc.). Preferably, the immune cell is a T cell or an NK cell, more preferably a T cell. The T cell also may be concentrated or purified. T cells may be at any developmental stage, including but not limited to: a CD4+CD8+ T cell, a CD4+ T cell (for example, Th1 and Th2 cells), CD8+ T cell (for example, cytotoxic T cell), a CD4-CD8- T cell, a tumor infiltrating cell, a memory T cell, a naive T cell, a γδ-T cell, an αβ-T cell, etc. Preferably, the immune cell is a human T cell, which can be obtained using a variety of techniques known to those skilled in the art, such as isolating T cells from the blood of a subject through Ficoll.

In some other embodiments, the cell is a stem cell.

The term "stem cell" refers to a primitive cell with the potential for self-replication, multilineage differentiation and homing. It is the origin cell of an organism, and the ancestral cell that gives rise to various tissues and organs of the human body. During the process of cell differentiation, the uneven distribution of regulatory differentiation proteins in the cytoplasm causes one daughter cell irreversibly proceeds toward terminal differentiation, becoming a differentiated cell with specific function until it completely loses the ability to divide again, eventually leading to cellular senescence and death. To compensate for this deficiency, the organism retains a portion of undifferentiated primitive cells, which preserve the parental characteristics. This portion of the retained undifferentiated primitive cells is called stem cells, and when needed, these stem cells can generate differentiated cells by division along developmental routes. The stem cell described in the present invention can be an embryonic stem cell, an adult stem cell (e.g., an umbilical cord blood stem cell, a bone marrow stem cell, a hematopoietic stem cell, a mesenchymal stem cell, etc.), and a pluripotent stem cell (e.g., an induced pluripotent stem cell iPSC, etc.).

In some embodiments, the expression of the endogenous HLA class I genes and/or HLA class II genes of the engineered cell of the present invention is not modified. That is, the expression level of any one of the endogenous HLA class I gene and/or HLA class II gene is not altered by any artificial intervention method (gene editing or non-gene editing).

In some embodiments, the expression of at least one of the endogenous HLA class I gene of the engineered cell of the present invention is suppressed or silenced. In some embodiments, the expression of at least one of the endogenous HLA class II gene of the engineered cell of the present invention is suppressed or silenced. In some embodiments, the expression of at least one of the endogenous TCR/CD3 gene of the engineered cell of the present invention is suppressed or silenced. In some embodiments, the expression of at least one of the endogenous TCR/CD3 gene and at least one of the endogenous HLA class I gene of the engineered cell of the present invention is suppressed or silenced. In some embodiments, the expression of at least one of the endogenous HLA class I gene and HLA class II gene of the engineered cell of the present invention is suppressed or silenced. In some embodiments, the expression of at least one of the endogenous TCR/CD3 gene, at least one of the endogenous HLA class I gene and at least one of the endogenous HLA class II gene of the engineered cell of the present invention is suppressed or silenced. Preferably, the HLA class I gene is selected from HLA-A, HLA-B, HLA-C and B2M. Preferably, the HLA class II gene is selected from HLA-DPA, HLA-DQ, HLA-DRA, TAP1, TAP2, LMP2, LMP7, RFX5, RFXAP, RFXANK and CIITA, preferably selected from RFX5, RFXAP, RFXANK and CIITA. Preferably, the TCR/CD3 gene is selected from TRAC, TRBC, CD3γ, CD3δ, CD3ε and CD3ζ.

In some embodiments, the expression of one or more endogenous genes of the engineered cell of the present invention is suppressed or silenced, the endogenous genes selected from the group consisting of: CD52, GR, dCK and immune checkpoint genes, e.g., PD1, LAG3, TIM3, CTLA4, PPP2CA, PPP2CB, PTPN6, PTPN22, PDCD1, HAVCR2, BTLA, CD160, TIGIT, CD96, CRTAM, TNFRSF10B, TNFRSF10A, CASP8, CASP10, CASP3, CASP6, CASP7, FADD, FAS, TGFBRII, TGFRBRI, SMAD2, SMAD3, SMAD4, SMAD10, SKI, SKIL, TGIF1, IL10RA, IL10RB, HMOX2, IL6R, IL6ST, EIF2AK4, CSK, PAG1, SIT, FOXP3, PRDM1, BATF, GUCY1A2, GUCY1A3, GUCY1B2, and GUCY1B3.

Methods for suppressing gene expression or silencing gene are well known to those skilled in the art, including but not limited to, for example, mediating DNA or RNA fragmentation by meganucleases, zinc finger nucleases, TALEN, CRISPR/Cas system, base editors, or inactivating genes through technologies, e.g., antisense oligonucleotides, RNAi, shRNA, transposons, mutaions, etc.

In some embodiments, the engineered cell of the present invention is an allogeneic cell. As used herein, the term "allogeneic" refers to any material derived from a different animal or a different patient of the same species as the individual into whom the material is introduced. Two or more individuals are considered allogeneic to each other when the genes at one or more loci differ. In some cases, allogeneic material from individuals of the same species may be genetically different enough for antigenic interactions to occur.

### Pharmaceutical composition

The present invention further provides a pharmaceutical composition, which comprises the engineered cell of the present invention as an active agent, and one or more pharmaceutically acceptable excipients. Therefore, the present invention further encompasses use of the engineered cell in the preparation of a pharmaceutical composition or a medicament.

The term "pharmaceutically acceptable excipient" refers to a vector and/or excipient that is pharmacologically and/or physiologically compatible (i.e., capable of triggering a desired therapeutic effect without causing any undesired local or systemic effects) with the subject and active ingredient, and it is well known in the art (see, e.g., Remington's Pharmaceutical Sciences. Edited by Gennaro AR, 19th ed. Pennsylvania: Mack Publishing Company, 1995). Examples of pharmaceutically acceptable excipient include, but are not limited to, filler, binder, disintegrant, coating agent, adsorbent, anti-adherent, glidant, antioxidant, flavoring agent, colorant, sweetener, solvent, co-solvent, buffer agent, chelating agent, surfactant, diluent, wetting agent, preservative, emulsifier, cladding agent, isotonic agent, absorption delaying agent, stabilizer, and tension regulator. It is known to those skilled in the art to select a suitable excipient to prepare the desired pharmaceutical composition of the present invention. Exemplary excipients for use in the pharmaceutical composition of the present invention include saline, buffered saline, dextrose, and water. Generally, the selection of a suitable excipient depends, in particular, on the active agent used, the disease to be treated, and the desired dosage form of the pharmaceutical composition.

The pharmaceutical composition according to the present invention is suitable for multiple routes of administration. Generally, the administration is parenterally accomplished. Parenteral delivery methods include topical, intraarterial, intramuscular, subcutaneous, intramedullary, intrathecal, intraventricular, intravenous, intraperitoneal, intrauterine, intravaginal, sublingual, or intranasal administration.

The pharmaceutical composition according to the present invention may also be prepared in various forms, e.g., solid, liquid, gaseous or lyophilized forms, and particularly may be in a form of ointment, cream, transdermal patch, gel, powder, tablet, solution, aerosol, granule, pill, suspension, emulsion, capsule, syrup, elixir, extract, tincture or liquid extract, or in a form particularly suitable for the desired method of administration. Processes known in the present invention for producing a medicament may include, for example, conventional mixing, dissolving, granulating, sugar-coating, grinding, emulsifying, encapsulating, embedding or lyophilizing process. The pharmaceutical composition comprising, for example, the immune cell as described herein is generally provided in a form of solution, and preferably comprises a pharmaceutically acceptable buffer agent.

The pharmaceutical composition according to the present invention further may be administered in combination with one or more other agents (biological agents, e.g., antibody reagents, and/or small molecules) or treatment methods (e.g., surgery, chemotherapy or radiotherapy) suitable for the treatment and/or prevention of diseases to be treated. Preferred examples of an agent suitable for the combination include known anti-cancer medicaments, e.g., cisplatin, maytansine derivatives, rachelmycin, calicheamicin, docetaxel, etoposide, gemcitabine, ifosfamide, irinotecan, melphalan, mitoxantrone, sorfimer sodium photofrin II, temozolomide, topotecan, trimetreate glucuronate, auristatin E, vincristine and doxorubicin; peptide cytotoxins, e.g., ricin, diphtheria toxin, pseudomonas exotoxin A, DNase and RNase; radionuclides, e.g., iodine 131, rhenium 186, indium 111, yttrium 90, bismuth 210, bismuth 213, actinium 225 and astatine 213; prodrugs, e.g., antibody-directed enzyme prodrugs; immunostimulatory agents, e.g., platelet factor 4, melanoma growth stimulating protein, etc.; antibodies or fragments thereof, e.g., anti-CD3 antibodies or fragments thereof, complement activators, heterologous protein domains, homologous protein domains, viral/bacterial protein domains and viral/bacterial peptides. In addition, the pharmaceutical composition of the present invention also can be used in combination with one or more other treatment methods, for example, chemotherapy and radiotherapy.

### Use and treatment methods

The present invention further provides use of the pharmaceutical composition or the engineered cell described above in the preparation of a medicament for diseases such as cancer, infection or autoimmune disease, and a method for treating cancer, infection or autoimmune diseases.

In some embodiments, the cancer is cancer associated with the expression of a target binding to a functional exogenous receptor. For example, the cancer includes, but is not limited to, brain glioma, blastoma, sarcoma, leukemia, basal cell carcinoma, biliary tract cancer, bladder cancer, bone cancer, brain and CNS cancer, breast cancer, peritoneal cancer, cervical cancer, choriocarcinoma, colon and rectal cancer, connective tissue cancer, cancer of the digestive system, endometrial cancer, esophageal cancer, eye cancer, head and neck cancer, gastric cancer (including gastrointestinal cancer), glioblastoma (GBM), liver cancer, hepatoma, intraepithelial neoplasia, kidney cancer, laryngeal cancer, liver tumor, lung cancer (e.g., small cell lung cancer, non-small cell lung cancer, adenocarcinoma of the lung, and squamous cell lung cancer), lymphoma (including Hodgkin's lymphoma and non-Hodgkin's lymphoma), melanoma, myeloma, neuroblastoma, oral cancer (e.g., lip, tongue, mouth, and pharynx), ovarian cancer, pancreatic cancer, prostate cancer, retinoblastoma, rhabdomyosarcoma, rectal cancer, cancer of the respiratory system, salivary gland cancer, skin cancer, squamous cell carcinoma, gastric cancer, testicular cancer, thyroid cancer, uterine or endometrial cancer, malignancy of the urinary system, vulvar cancer, and other cancers and sarcomas, and B-cell lymphoma (including low-grade/follicular non-Hodgkin's lymphoma (NHL), small lymphocytic (SL) NHL, intermediate-grade/follicular NHL, intermediate-grade diffuse NHL, high-grade immunoblastic NHL, high-grade lymphoblastic NHL, high-grade small non-cleaved cell NHL, large mass disease NHL), mantle cell lymphoma, AIDS-related lymphoma, and Waldenstrom macroglobulinemia, chronic lymphocytic leukemia (CLL), acute lymphoblastic leukemia (ALL), B-cell acute lymphoblastic leukemia (B-ALL), T-cell acute lymphoblastic leukemia (T-ALL), B-cell prolymphocytic leukemia, blastic plasmacytoid dendritic cell neoplasm, Burkitt's lymphoma, diffuse large B-cell lymphoma, follicular lymphoma, chronic myeloid leukemia (CML), malignant lymphoproliferative disease, MALT lymphoma, hairy cell leukemia, marginal zone lymphoma, multiple myeloma, myelodysplasia, plasmablastic lymphoma, preleukemia, plasmacytoid dendritic cell neoplasm, and post-transplant lymphoproliferative disorder (PTLD); and other diseases associated with target expression. Preferably, the disease that can be treated with the engineered cell or the pharmaceutical composition of the present invention is selected from the group consisting of: leukemia, lymphoma, multiple myeloma, brain glioma, pancreatic cancer, gastric cancer, etc.

In some embodiments, the infection includes, but is not limited to, infections caused by viruses, bacteria, fungi, and parasites.

In some embodiments, the autoimmune disease includes, but is not limited to type I diabetes, celiac disease, Graves' disease, inflammatory bowel disease, multiple sclerosis, psoriasis, rheumatoid arthritis, Addison's disease, Sjögren's syndrome, Hashimoto's thyroiditis, myasthenia gravis, vasculitis, pernicious anemia and systemic lupus erythematosus.

The present invention will be described in detail below with reference to the accompanying drawings and examples. It should be noted that those skilled in the art should understand that the drawings of the present invention and the examples thereof are only for the purpose of illustration, and shall not constitute any limitation to the present invention. In the case of no contradiction, the embodiments in the present invention and the features in the embodiments can be combined with each other.

### BRIEF DESCRIPTION OF DRAWINGS

Figure 1: CAR expression levels in CAR19 T, CAR19-P28A T and CAR19-PNRA T cells.
Figure 2: Anti-NKG2A antibody expression levels in CAR19 T, CAR19-P28A T and CAR19-PNRA T cells.
Figure 3: PD-L1 expression levels in CAR19 T, CAR19-P28A T, and CAR19-PNRA T cells.
Figure 4: In vitro killing effect of CAR19 T, CAR19-P28A T and CAR19-PNRA T cells against target cells.
Figure 5: Cytokine release levels of CAR19 T, CAR19-P28A T, and CAR19-PNRA T cells after co-incubation with target cells.
Figure 6: In vivo tumor inhibition effects of CAR19 T, CAR19-P28A T and CAR19-PNRA T cells.
Figure 7: In vitro killing effects of CAR19-PNR T, CAR19-PNRC4 T, CAR19-PNRT3 T, CAR19-PNRL3 T and CAR19-PNRTT T cells against target cells.
Figure 8: Cytokine release levels in CAR19-PNR T, CAR19-PNRC4 T, CAR19-PNRT3 T, CAR19-PNRL3 T and CAR19-PNRTT T cells after co-incubation with target cells.

### DETAILED DESCRIPTION

### Example 1: Construction of anti-CD19 CAR-T cells expressing PD-L1 and NKG2A binding molecules and verification of the function thereof

Sequences encoding the following were synthesized and sequentially cloned into the pGEM-T Easy vector (Promega): anti-CD19 scFv (SEQ ID NO: 105), CD8α hinge region (SEQ ID NO: 86), CD8α transmembrane region (SEQ ID NO: 83), 4-1BB co-stimulatory domain (SEQ ID NO: 90), CD3ζ intracellular region (SEQ ID NO: 92) to obtain a CAR19 plasmid, and the correct insertion of the target sequence in the plasmid was confirmed by sequencing.

A PD1-binding molecule and an NKG2A binding molecule (linked by a 2A peptide) were further comprised in the CAR19 plasmid prepared in Example 1, wherein the structure of the PD1-binding molecule was as follows: PD-L1 signal peptide (SEQ ID NO:164), PD-L1 extracellular domain (SEQ ID NO:78), and PD-L1 transmembrane domain (SEQ ID NO:80) and the structure of the NKG2A binding molecule was as follows: B2M signal peptide (SEQ ID NO:94), NKG2A binding domain (SEQ ID NO:9), IgG4 hinge region (SEQ ID NO:88), CD28 transmembrane region (SEQ ID NO:82), and CD28 co-stimulatory domain (SEQ ID NO:89), to obtain a CAR19-PNRA plasmid, and the correct insertion of the target sequence in the plasmid was confirmed by sequencing.

A CD28 co-stimulatory domain (SEQ ID NO:89) was further comprised in the PD1-binding molecule of the CAR19-PNRA plasmid to obtain a CAR19-P28A plasmid, and the correct insertion of the target sequence in the plasmid was confirmed by sequencing.

### 1.1 Construction of CAR19-PNRA T cells

Three ml Opti-MEM (Gibco) was added to a sterile tube to dilute the CAR19, CAR19-P28A, CAR19-PNRA plasmids, respectively, and then packaging vector psPAX2 (Addgene) and envelope vector pMD2.G (Addgene) were added according to the ratio of plasmid : viral packaging vector : viral envelope vector = 4:2:1. Then, 120 µl X-treme GENE HP DNA transfection reagent (Roche) was added, mixed immediately, and incubated at room temperature for 15 min, and then the plasmid/vector/transfection reagent mixture was added dropwise to the culture flask of 293T cells. Viruses were collected at 24 hours and 48 hours, pooled, and ultracentrifuged (25000 g, 4°C, 2.5 hours) to obtain concentrated lentiviruses.

T cells were activated with DynaBeads CD3/CD28 CTSTM (Gibco), and were further cultured for 1 day under 37°C and 5% CO₂. On the second day, the concentrated lentiviruses were added, and after 3 days of continuous culture, T cells expressing CAR19 (and PD1-binding molecules and NKG2A binding molecules) (CAR19 T, CAR19-P28A T, and CAR19-PNRA T cells) were obtained.

### 1.2 Detection of CAR expression levels and PD1/NKG2A binding domain expression levels in various CAR-T cells

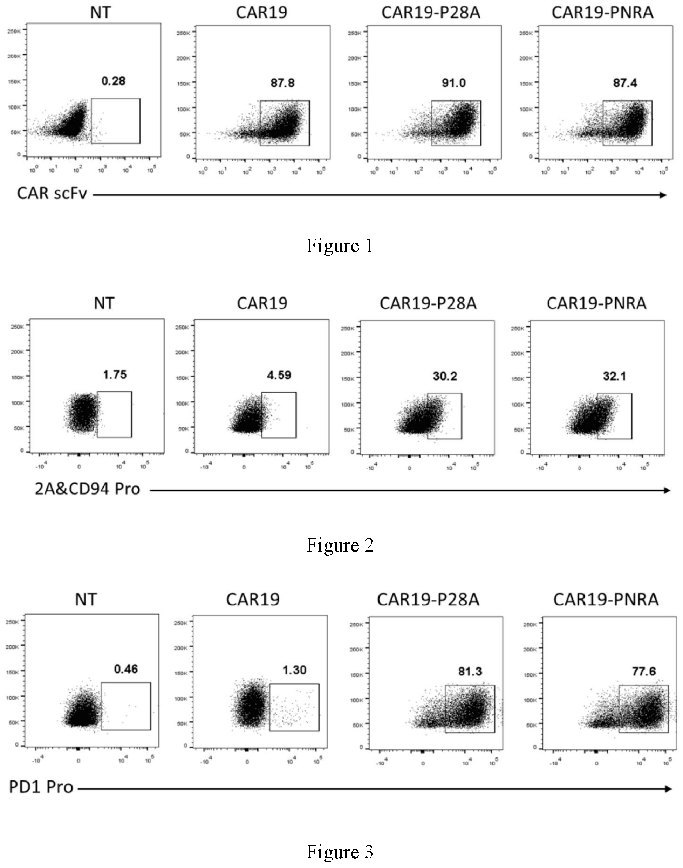

Using a flow cytometry, the expression of anti-CD19 scFv in CAR19 T, CAR19-P28A T, and CAR19-PNRA T cells was detected with FITC goat anti-human Fab (Thermo Fisher). It can be seen that all the CARs in the CAR-T cells prepared in this example can be effectively expressed (Figure 1).

Using a flow cytometry, the expression of anti-NKG2A scFv in CAR19-P28A T cells and CAR19-PNRA T cells was detected with NKG2A&CD94 Protein (ACRO), and the expression of PDL1 in CAR19-P28A T and CAR19-PNRA T cells was detected with PD1 Protein (ACRO). It can be seen that anti-NKG2A scFv and PDL1 in CAR19-P28A T cells and CAR19-PNRA T cells were effectively expressed (Figures 2 and 3).

### 1.3 Detection of the in vitro killing effects of various CAR-T cells on target cells

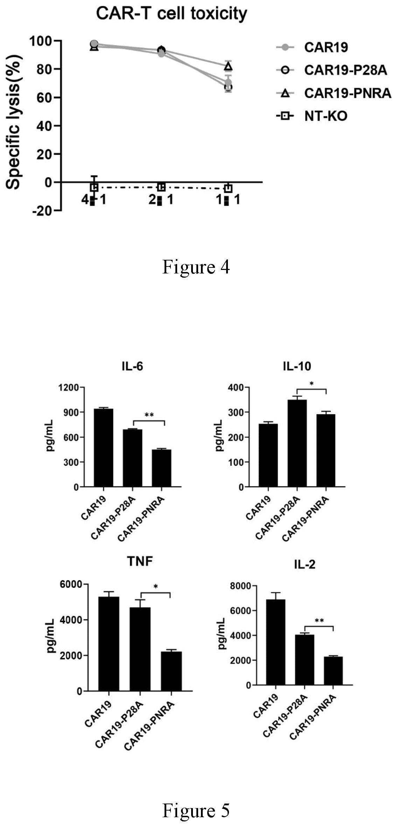

To detect the killing ability of CAR-T cells against target cells, Raji target cells carrying luciferase gene were first plated into 96-well plates at 1×10⁴ cells/well, and then, three types of CAR T cells and NT cells were plated into the 96-well plates at effector-to-target ratios of 4 :1, 2:1, and 1:1, respectively, for co-culture. After 8 hours, the fluorescence value was measured with a microplate reader. According to the calculation formula: (average fluorescence value of target cells - average fluorescence value of samples)/average fluorescence value of target cells × 100%, the killing efficiency was calculated, and the results are shown in Figure 4.

It can be seen that at effector-to-target ratios of 4:1 and 2:1, there is no significant difference in the in vitro killing effect of CAR19 T, CAR19-P28A T and CAR19-PNRA T cells on target cells; at an effector-to-target ratio of 1:1, CAR19-PNRA T cells show a better in vitro killing effect on target cells than CAR19 T and CAR19-P28A T cells.

### 1.4 Detection of cytokine release levels in various CAR-T cells

Considering that the intracellular CD28 co-activation signal would lead to rapid amplification of CAR-T cells in vivo, thereby increasing the risk of CRS in patients, the inventors thus investigated the secretion of CRS-related cytokines by two types of CAR-T cells under the condition where both macrophages and tumor cells are present.

PBMCs from the same donor as CAR-T were resuscitated, and 20 ng/mL M-CSF (PEPROTECH) was added to induce macrophage differentiation. After 7 days of induction culture, they were used for subsequent experiments.

First, the induced differentiated macrophages were plated in 96-well plates at a concentration of 2×10⁴ cells/well, then target cells Raji were plated in 96-well plates at a concentration of 1×10⁵ cells/well, and finally, CAR19 T cells, CAR19-P28A T cells, CAR19-PNRA T cells, and NT cells were added at a target cell : CAR-T ratio of 1:1, respectively; the cell co-culture supernatant was collected after 18-24 hours of co-culture.

Human IL-6 DuoSet ELISA Kit (R&D systems), Human IL-10 DuoSet ELISA Kit (R&D systems), Human IL-2 DuoSet ELISA Kit (R&D systems), and Human IFN-gamma DuoSet ELISA Kit (R&D systems) were used to detect the contents of IL6, IL10, IL2 and IFN-γ in the co-culture supernatant, respectively, and the results are shown in Figure 5.

As can be seen, CAR19-PNRA T cells can significantly reduce the secretion of CRS-related cytokines (IL6, IL10, IL2, and IFN-γ) compared with CAR19 T and CAR19-P28A T. This indicates that CAR19-PNRA T cells can reduce the risk of CRS in patients.

### 1.5 Detection of in vivo suppressive effect of various CAR-T cells on tumors

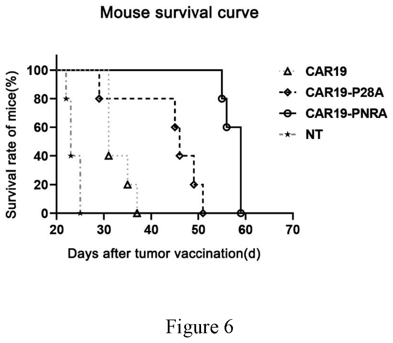

Fifteen healthy female NPI mice aged 6-8 weeks were divided into 3 groups, with 5 mice in each group: CAR19 T cell group, CAR19-P28A T cell group, and CAR19-PNRA T cell group. On day 0, 5×10⁵ Raji cells were injected into the tail vein of each mouse. Six days later, 2×10⁶ corresponding CAR-T cells were injected into the tail vein of each mouse according to the grouping. The status of the mice was assessed weekly. The mouse survival curve is shown in Figure 6.

As can be seen, the CAR19-PNRA T cell group significantly improves the survival rate of mice and shows a significant tumor-suppressing effect compared with the CAR19 T cell group and the CAR19-P28A T cell group. This indicates that the CAR19-PNRA T cells prepared in the present invention can enhance the in vivo killing activity of CAR-T cells.

### Example 2: Construction of anti-CD19 CAR-T cells expressing PD-L1 and CTLA4/TIM3/LAG3/TIGIT binding molecules and verification of the function thereof

A PD1-binding molecule (linked by a 2A peptide) was further comprised in the CAR19 plasmid prepared in Example 1, wherein the structure of the PD1-binding molecule was as follows: PD-L1 signal peptide (SEQ ID NO:164), PD-L1 extracellular domain (SEQ ID NO:78), and PD-L1 transmembrane domain (SEQ ID NO:80), to obtain a CAR19-PNR plasmid, and the correct insertion of the target sequence in the plasmid was confirmed by sequencing.

A CTLA4 binding molecule, a TIM3 binding molecule, a LAG3 binding molecule, or a TIGIT binding molecule (linked by a 2A peptide) was further comprised in the CAR19-PNR plasmid prepared in Example 2 to obtain CAR19-PNRC4, CAR19-PNRT3, CAR19-PNRL3, and CAR19-PNRTT plasmids, respectively, and the correct insertion of the target sequence in the plasmids was confirmed by sequencing.

The structure of the CTLA4 binding molecule was as follows: B2M signal peptide (SEQ ID NO:94), CTLA4 binding domain (SEQ ID NO:64), IgG4 hinge region (SEQ ID NO:88), CD28 transmembrane region (SEQ ID NO:82), and CD28 co-stimulatory domain (SEQ ID NO:89).

The structure of the TIM3 binding molecule was as follows: B2M signal peptide (SEQ ID NO:94), TIM3 binding domain (SEQ ID NO:21), IgG4 hinge region (SEQ ID NO:88), CD28 transmembrane region (SEQ ID NO:82), and CD28 co-stimulatory domain (SEQ ID NO:89).

The structure of the LAG3 binding molecule was as follows: B2M signal peptide (SEQ ID NO:94), LAG3 binding domain (SEQ ID NO:34), IgG4 hinge region (SEQ ID NO:88), CD28 transmembrane region (SEQ ID NO:82), and CD28 co-stimulatory domain (SEQ ID NO:89).

The structure of the TIGIT binding molecule was as follows: B2M signal peptide (SEQ ID NO:94), TIGIT binding domain (SEQ ID NO:47), IgG4 hinge region (SEQ ID NO:88), CD28 transmembrane region (SEQ ID NO:82), and CD28 co-stimulatory domain (SEQ ID NO:89), to obtain a CAR19-PNRTT plasmid, and the correct insertion of the target sequence in the plasmid was confirmed by sequencing.

### 2.1 Construction of CAR T cells

Three ml Opti-MEM (Gibco) was added to a sterile tube to dilute the CAR19-PNR, CAR19-PNRC4, CAR19-PNRT3, CAR19-PNRL3, and CAR19-PNRTT plasmids, respectively, and then packaging vector psPAX2 (Addgene) and envelope vector pMD2.G (Addgene) were added according to the ratio of plasmid : viral packaging vector : viral envelope vector = 4:2:1. Then, 120 µl X-treme GENE HP DNA transfection reagent (Roche) was added, mixed immediately, and incubated at room temperature for 15 min, and then the plasmid/vector/transfection reagent mixture was added dropwise to the culture flask of 293T cells. Viruses were collected at 24 hours and 48 hours, pooled, and ultracentrifuged (25000 g, 4°C, 2.5 hours) to obtain concentrated lentiviruses.

T cells were activated with DynaBeads CD3/CD28 CTSTM (Gibco), and were further cultured for 1 day under 37°C and 5% CO₂. On the second day, the concentrated lentiviruses were added, and after 3 days of continuous culture, T cells expressing CAR19-PNR, CAR19-PNRC4, CAR19-PNRT3, CAR19-PNRL3, and CAR19-PNRTT (CAR19-PNR T, CAR19-PNRC4 T, CAR19-PNRT3 T, CAR19-PNRL3 T, and CAR19-PNRTT T cells) were obtained.

### 2.2 Detection of the in vitro killing effects of various CAR-T cells on target cells

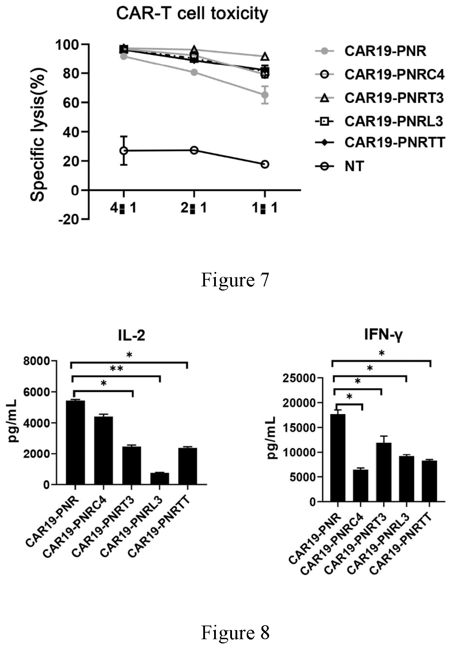

To detect the killing ability of CAR-T cells against target cells, Raji target cells carrying luciferase gene were first plated into 96-well plates at 1×10⁴ cells/well, and then, 5 types of CAR T cells and NT cells were plated into the 96-well plates at effector-to-target ratios of 4 :1, 2:1, and 1:1, respectively, for co-culture. After 8 hours, the fluorescence value was measured with a microplate reader. According to the calculation formula: (average fluorescence value of target cells - average fluorescence value of samples)/average fluorescence value of target cells × 100%, the killing efficiency was calculated, and the results are shown in Figure 7.

It can be seen that at an effector-to-target ratio of 4:1, there is no significant difference in the in vitro killing effects of various CAR T cells on target cells; at effector-to-target ratios of 2:1 and 1:1, CAR19-PNRC4 T, CAR19-PNRT3 T, CAR19-PNRL3 T and CAR19-PNRTT T cells show a better in vitro killing effect on target cells than CAR19-PNR T cells.

### 2.3 Detection of cytokine release levels in various CAR-T cells

PBMCs from the same donor as CAR-T were resuscitated, and 20 ng/mL M-CSF (PEPROTECH) was added to induce macrophage differentiation. After 7 days of induction culture, they were used for subsequent experiments.

First, the induced differentiated macrophages were plated in 96-well plates at a concentration of 2×10⁴ cells/well, then target cells Raji were plated in 96-well plates at a concentration of 1×10⁵ cells/well, and finally, CAR19-PNR, CAR19-PNRC4, CAR19-PNRT3, CAR19-PNRL3, CAR19-PNRTT cells and NT cells were added at a target cell : CAR-T ratio of 1:1, respectively; the cell co-culture supernatant was collected after 18-24 hours of co-culture.

Human IL-2 DuoSet ELISA Kit (R&D systems) and Human IFN-gamma DuoSet ELISA Kit (R&D systems) were used to detect the contents of IL2 and IFN-γ in the co-culture supernatant, respectively, and the results are shown in Figure 8.

It can be seen that, CAR19-PNRC4 T, CAR19-PNRT3 T, CAR19-PNRL3 T, and CAR19-PNRTT T cells can significantly reduce the secretion of cytokines (IL2 and IFN-γ) compared with CAR19-PNR T. This indicates that CAR19-PNRC4 T, CAR19-PNRT3 T, CAR19-PNRL3 T, and CAR19-PNRTT T cells can reduce the risk of CRS in patients compared with CAR19-PNR T cells.

In conclusion, the simultaneous expression of PD1-binding molecules and immunosuppressive molecules would not adversely affect the in vitro killing activity of CAR-T cells, and can significantly reduce the risk of CRS in patients and enhance the in vivo tumor-suppressive effect of CAR-T cells.

It should be noted that the above-mentioned are merely preferred examples of the present invention and not to limit the present invention. For those skilled in the art, various modifications and changes may be made to the present invention. Those skilled in the art should understand that any amendments, equivalent replacements, improvements, and so on, made within the spirit and principle of the present invention, should be covered within the scope of protection of the present invention.

## Claims

1. An engineered cell, which expresses:
(i) a PD1-binding molecule comprising a PD1-binding domain and a transmembrane domain and not comprising any intracellular domain other than an intracellular domain derived from a PD1 ligand;
(ii) an immunosuppressive molecule comprising an immunosuppressive protein binding domain and a transmembrane domain and not comprising a primary signaling domain, wherein the immunosuppressive protein is selected from NKG2A, TIM3, LAG3, TIGIT, CTLA4, IRP60, SIRPα, KIR2DL1/2/3, LILRB1 or a combination thereof.

2. The engineered cell of claim 1, **characterized in that** the binding domain is selected from the group consisting of an intact antibody, Fab, Fab', F(ab')2, an Fd fragment, Fd', an Fv fragment, scFv, sdFv, a linear antibody, a diabody, sdAb, and a functional fragment of a ligand or a receptor.

3. The engineered cell of claim 1 or 2, **characterized in that** the PD1 binding domain is selected from the group consisting of a PD1-targeting antibody, a PD1-binding ligand, or a functional fragment thereof.

4. The engineered cell of claim 3, **characterized in that** the PD1-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 75; and the CDR1-L, CDR2-L and CDR3-L comprised in the light chain variable region are the same as the CDR1-L, CDR2-L and CDR3-L comprised in SEQ ID NO: 76; preferably, the heavy chain variable region comprises an amino acid sequence having at least 90% identity to SEQ ID NO: 75, and the light chain variable region comprises an amino acid sequence having at least 90% identity to SEQ ID NO: 76, and preferably, the PD1 binding domain comprises an amino acid sequence having at least 90% identity to SEQ ID NO:77.

5. The engineered cell of claim 3, **characterized in that** the PD1-targeting ligand comprises an amino acid sequence having at least 90% identity to SEQ ID NO: 78 or 79.

6. The engineered cell of any one of claims 1-5, **characterized in that** the immunosuppressive molecule comprises an NKG2A binding domain, a TIM3 binding domain, an LAG3 binding domain, a TIGIT binding domain, a CTLA4 binding domain, an IRP60 binding domain, an SIRPα binding domain, a KIR2DL1/2/3 binding domain, an LILRB1 binding domain, or a combination thereof; wherein,
(1) the NKG2A binding domain is an NKG2A-targeting antibody, an NKG2A-binding ligand, or a functional fragment thereof;
(2) the TIM3 binding domain is a TIM3-targeting antibody, a TIM3-binding ligand, or a functional fragment thereof;
(3) the LAG3 binding domain is an LAG3-targeting antibody, an LAG3-binding ligand, or a functional fragment thereof;
(4) the TIGIT binding domain is a TIGIT-targeting antibody, a TIGIT -binding ligand, or a functional fragment thereof;
(5) the CTLA4 binding domain is a CTLA4-targeting antibody, a CTLA4-binding ligand, or a functional fragment thereof;
(6) the IRP60 binding domain is an IRP60-targeting antibody, an IRP60-binding ligand, or a functional fragment thereof;
(7) the SIRPα binding domain is an SIRPα-targeting antibody, an SIRPα-binding ligand, or a functional fragment thereof;
(8) the KIR2DL1/2/3 binding domain is a KIR2DL1/2/3-targeting antibody, a KIR2DL1/2/3-binding receptor, or a functional fragment thereof; or
(9) the LILRB1 binding domain is an LILRB1-targeting antibody, an LILRB1-binding receptor, or a functional fragment thereof.

7. The engineered cell of claim 6, **characterized in that**:
(1) the NKG2A-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 7; and the CDR1-L, CDR2-L and CDR3-L comprised in the light chain variable region are the same as the CDR1-L, CDR2-L and CDR3-L comprised in SEQ ID NO: 8; preferably, the heavy chain variable region comprises an amino acid sequence having at least 90% identity to SEQ ID NO:7, and the light chain variable region comprises an amino acid sequence having at least 90% identity to SEQ ID NO:8;
(2) the TIM3-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 19; and the CDR1-L, CDR2-L and CDR3-L comprised in the light chain variable region are the same as the CDR1-L, CDR2-L and CDR3-L comprised in SEQ ID NO: 20; preferably, the heavy chain variable region comprises an amino acid sequence having at least 90% identity to SEQ ID NO: 19, and the light chain variable region comprises an amino acid sequence having at least 90% identity to SEQ ID NO: 20;
(3) the LAG3-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 32; and the CDR1-L, CDR2-L and CDR3-L comprised in the light chain variable region are the same as the CDR1-L, CDR2-L and CDR3-L comprised in SEQ ID NO: 33; preferably, the heavy chain variable region comprises an amino acid sequence having at least 90% identity to SEQ ID NO: 32, and the light chain variable region comprises an amino acid sequence having at least 90% identity to SEQ ID NO: 33;
(4) the TIGIT-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 45; and the CDR1-L, CDR2-L and CDR3-L comprised in the light chain variable region are the same as the CDR1-L, CDR2-L and CDR3-L comprised in SEQ ID NO: 46; preferably, the heavy chain variable region comprises an amino acid sequence having at least 90% identity to SEQ ID NO: 45, and the light chain variable region comprises an amino acid sequence having at least 90% identity to SEQ ID NO: 46;
(5) the CTLA4-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 62; and the CDR1-L, CDR2-L and CDR3-L comprised in the light chain variable region are the same as the CDR1-L, CDR2-L and CDR3-L comprised in SEQ ID NO: 63; preferably, the heavy chain variable region comprises an amino acid sequence having at least 90% identity to SEQ ID NO: 62, and the light chain variable region comprises an amino acid sequence having at least 90% identity to SEQ ID NO: 63;
(6) the IRP60-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 150; and the CDR1-L, CDR2-L and CDR3-L comprised in the light chain variable region are the same as the CDR1-L, CDR2-L and CDR3-L comprised in SEQ ID NO: 151; preferably, the heavy chain variable region comprises an amino acid sequence having at least 90% identity to SEQ ID NO: 150, and the light chain variable region comprises an amino acid sequence having at least 90% identity to SEQ ID NO: 151;
(7) the SIRPα-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 159; and the CDR1-L, CDR2-L and CDR3-L comprised in the light chain variable region are the same as the CDR1-L, CDR2-L and CDR3-L comprised in SEQ ID NO: 160; preferably, the heavy chain variable region comprises an amino acid sequence having at least 90% identity to SEQ ID NO: 159, and the light chain variable region comprises an amino acid sequence having at least 90% identity to SEQ ID NO: 160;
(8) the KIR2DL1/2/3-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 119 or 122; wherein the CDR1-L, CDR2-L and CDR3-L comprised in the light chain variable region are the same as the CDR1-L, CDR2-L and CDR3-L comprised in SEQ ID NO: 120 or 123; preferably, the heavy chain variable region comprises an amino acid sequence having at least 90% identity to SEQ ID NO: 119 or 122, and the light chain variable region comprises an amino acid sequence having at least 90% identity to SEQ ID NO: 120 or 123; or
(9) the LILRB1-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 131 or 140; wherein the CDR1-L, CDR2-L and CDR3-L comprised in the light chain variable region are the same as the CDR1-L, CDR2-L and CDR3-L comprised in SEQ ID NO: 132 or 141; preferably, the heavy chain variable region comprises an amino acid sequence having at least 90% identity to SEQ ID NO: 131 or 140, and the light chain variable region comprises an amino acid sequence having at least 90% identity to SEQ ID NO: 132 or 141.

8. The engineered cell of claim 6, **characterized in that**:
(1) the NKG2A-targeting ligand comprises an amino acid sequence having at least 90% identity to SEQ ID NO: 10 or 11;
(2) the TIM3-targeting ligand comprises an amino acid sequence having at least 90% identity to SEQ ID NO: 22, 23 or 24;
(3) the LAG3-targeting ligand comprises an amino acid sequence having at least 90% identity to SEQ ID NO: 35, 36 or 37;
(4) the TIGIT-targeting ligand comprises an amino acid sequence having at least 90% identity to SEQ ID NO: 48, 49, 50 or 51;
(5) the CTLA4-targeting ligand comprises an amino acid sequence having at least 90% identity to SEQ ID NO: 65 or 66;
(6) the SIRPα-targeting ligand comprises an amino acid sequence having at least 90% identity to SEQ ID NO: 163; or
(7) the LILRB1-targeting ligand comprises an amino acid sequence having at least 90% identity to SEQ ID NO: 143.

9. The engineered cell of any one of claims 1-8, **characterized in that** the immunosuppressive molecule further comprises a co-stimulatory domain.

10. The engineered cell of any one of claims 1-9, **characterized in that** the engineered cell further expresses: (iii) a functional exogenous receptor that specifically recognizes an antigen.

11. The engineered cell of claim 10, **characterized in that** the functional exogenous receptor is selected from the group consisting of: a chimeric antigen receptor, a T cell receptor, a T cell receptor fusion protein, a T cell antigen coupler, and an immune mobilizing monoclonal T cell receptor, preferably a chimeric antigen receptor or a T cell receptor.

12. The engineered cell of claim 11, **characterized in that** the functional exogenous receptor is selected from a chimeric antigen receptor comprising an antigen binding domain, a transmembrane domain, and a primary signaling domain, optionally, the chimeric antigen receptor further comprising a co-stimulatory domain.

13. The engineered cell of claim 10, **characterized in that** the antigen specifically recognized by the functional exogenous receptor is selected from the group consisting of: ALK, ADRB3, AKAP-4, APRIL, ASGPR1, BCMA, B7H3, B7H4, B7H6, bcr-abl, BORIS, BST2, BAFF-R, BTLA, CD2, CD3, CD4, CD5, CD7, CD8, CD19, CD20, CD22, CD24, CD25, CD28, CD30, CD33, CD38, CD40, CD44, CD44v6, CD44v7/8, CD47, CD52, CD56, CD57, CD58, CD70, CD72, CD79a, CD79b, CD80, CD81, CD86, CD97, CD123, CD133, CD137, CD 138, CD151, CD171, CD179a, CD300LF, CDH16, CSPG4, CS1, Claudin 6, Claudin18.1, Claudin 18.2, CEA, CEACAM6, CLL1, c-Met, CAIX, CXORF61, CA125, CYP1B1, CS1, ELF2M, EGFR, EPCAM, EGFRvIII, EphA2, ERG/TMPRSS2ETS fusion gene, ETV6-AML, EMR2, EGP2, EGP40, FAP, FAR, FBP, FLT3, FOSL1, FCRL5, FCAR, Flt3, Flt4, Frizzled, GD2, GD3, gp100, gp130, GM3, GPC2, GPC3, GPRC5D, GPR20, GloboH, GHRHR, GHR, GITR, Her2, HER3, HER-4, HMWMAA, HAVCR1, HPV E6,E7, HVEM, HIV-1Gag, HLA-A1, HLA-A2, IL6R, IL-11Ra, IL-13Ra, IGF-I receptor, LTPR, LIFRP, LRP5, IGLL1, IGF1R, KIT, Kappa Light Chain, KDR, LewisY, LMP2, LY6K, LAGE-1a, legumain, LCK, LAIR1, LILRA2, LY75, MSLN, MUC1, MUC16, MAGE-A1, MAGE3, MAD-CT-1, MelanA/MART1, ML-IAP, MYCN, mut hsp70-2, NCAM, NY-BR-1, NY-ESO-1, NA17, Notch-1-4, nAchR, NKG2D, NKG2D ligand, OY-TES1, OR51E2, OX40, PRSS21, PSCA, PD1, PD-L1, PD-L2, PSMA, Prostase, PAP, PDGFR-β, PCTA-1/galectin 8, p53, p53 mutant, prostein, PLAC1, PANX3, PAX3, PAX5, PTCH1, RANK, RAGE-1, ROR1, Ras mutant, RhoC, RU1, RU2, Robol, SSEA-4, SSX2, SART3, Sp17, TSHR, Tn Ag, TGS5, TEM1/CD248, TEM7R, TARP, TCRα, TCRβ, TGFBR1, TGFBR2, TNFRSF4, TWEAK-R, TLR7, TLR9, TAG72, TROP-2, Tie 2, TRP-2, TNFR1, TNFR2, TEM1, UPK2VEGFR, WT1, XAGE1, 5T4, 8H9, αvβ6 integrin, CA9, folate receptor α, ephrin B2, tyrosinase, fucosyl GM1, o-acetyl-GD2, folate receptor β, polysialic acid, sperm protein 17, survivin and telomerase, sarcoma translocation breakpoint, human telomerase reverse transcriptase/hTERT, androgen receptor, intestinal carboxylesterase, cyclin B1, fibronectin, tenascin, carcinoembryonic variant of tumor necrosis region, and any combination thereof.

14. The engineered cell of any one of claims 1-13, **characterized in that** the transmembrane domain is selected from a transmembrane domain of a protein selected from the group consisting of: a TCR α chain, a TCR β chain, a TCR γ chain, a TCR δ chain, a CD3 ζ subunit, a CD3 ε subunit, a CD3 γ subunit, a CD3 δ subunit, CD28, CD45, CD4, CD5, CD8α, CD9, CD16, CD22, CD33, CD37, CD47, CD64, CD80, CD86, CD94, CD112, CD113, CD134, CD137, CD154, CD155, Nectin4, KIRDS2, OX40, CD2, CD27, CD18, ICOS, 4-1BB, GITR, CD40, BAFFR, HVEM, SLAMF7, NKp80, CD160, BCMA, IL-2Rβ, IL-2Rγ, IL-7Ra, ITGA1, VLA1, CD49a, ITGA4, IA4, CD49D, ITGA6, VLA-6, CD49f, ITGAD, CDl ld, ITGAE, CD103, ITGAL, CDl la, ITGAM, CDl lb, ITGAX, CDl lc, ITGB1, CD29, ITGB2, CD18, LFA-1, ITGB7, TNFR2, DNAM1, SLAMF4, CD84, CD96, LSECtin, PDL1, PDL2, CEACAM1, MHC class I molecules, MHC class II molecules, CRT AM, Ly9, CD160, PSGL1, CDIOO, SLAMF6, SLAMF1, SLAMF8, CD162, LTBR, PAG/Cbp, NKp44, NKp30, NKp46, NKG2D, and NKG2C.

15. The engineered cell of any one of claims 1-14, **characterized in that** the co-stimulatory domain is selected from the intracellular regions of the proteins from the group consisting of: LTB, CD94, TLR1, TLR2, TLR3, TLR4, TLR5, TLR6, TLR7, TLR8, TLR9, TLR10, CARD11, CD2, CD7, CD8, CD18, CD27, CD28, CD30, CD40, CD54, CD83, CD134, 4-1BB, CD270, CD272, B7-H3, ICOS, CD357, DAP10, DAP12, LAT, NKG2C, SLP76, PD1, LIGHT, TRIM, ZAP70, and any combination thereof.

16. The engineered cell of any one of claims 1-15, **characterized in that** the primary signaling domain is selected from the intracellular regions of the proteins from the group consisting of: FcRγ, FcRβ, CD3γ, CD3δ, CD3ε, CD3ζ, CD5, CD22, CD79a, CD79b, NFAM1, STAM1, STAM2, and CD66d.

17. The engineered cell of any one of claims 1-16, **characterized in that** a hinge region is further comprised between the binding domain and the transmembrane domain, and the hinge region is derived from the proteins from the group consisting of: CD8α, CD28, FcγRIIIα receptor, IgG4 or IgG1.

18. The engineered cell of any one of claims 1-17, **characterized in that** in the engineered cell, expression of one or more genes among endogenous HLA class I gene, HLA class II gene, and TCR/CD3 gene is suppressed or silenced.

19. The engineered cell of claim 18, **characterized in that** the HLA class I gene is selected from the group consisting of HLA-A, HLA-B, HLA-C, B2M and any combination thereof; the HLA class II gene is selected from the group consisting of HLA-DPA, HLA-DQ, HLA-DRA, TAP1, TAP2, LMP2, LMP7, RFX5, RFXAP, RFXANK, CIITA and any combination thereof; and the TCR/CD3 gene is selected from the group consisting of TRAC, TRBC, CD3γ, CD3δ, CD3ε, CD3ζ and any combination thereof.

20. The engineered cell of any one of claims 1-19, **characterized in that** expression of one or more endogenous genes selected from the following is suppressed or silenced: CD52, GR, dCK, PD1, LAG3, TIM3, CTLA4, PPP2CA, PPP2CB, PTPN6, PTPN22, PDCD1, HAVCR2, BTLA, CD160, TIGIT, CD96, CRTAM, TNFRSF10B, TNFRSF10A, CASP8, CASP10, CASP3, CASP6, CASP7, FADD, FAS, TGFBRII, TGFRBRI, SMAD2, SMAD3, SMAD4, SMAD10, SKI, SKIL, TGIF1, IL10RA, IL10RB, HMOX2, IL6R, IL6ST, EIF2AK4, CSK, PAG1, SIT, FOXP3, PRDM1, BATF, GUCY1A2, GUCY1A3, GUCY1B2, and GUCY1B3.

21. The engineered cell of any one of claims 1-20, **characterized in that** the cell is an immune cell, and the immune cell being selected from the group consisting of a B cell, a T cell, a macrophage, a dendritic cell, a monocyte, an NK cell, and an NKT cell.

22. The engineered cell of claim 21, **characterized in that** the cell is selected from the group consisting of a CD4+CD8+ T cell, a CD4+ T cell, a CD8+ T cell, a CD4-CD8- T cell, a tumor infiltrating cell, a memory T cell, a naive T cell, a γδ-T cell and an αβ-T cell.

23. The engineered cell of claim 21, **characterized in that** the cell is a stem cell, the stem cell being selected from the group consisting of an embryonic stem cell, an umbilical cord blood stem cell, a bone marrow stem cell, a hematopoietic stem cell, a mesenchymal stem cell and an induced pluripotent stem cell.

24. A pharmaceutical composition comprising the engineered cell of any one of claims 1-23, and one or more pharmaceutically acceptable excipients.

25. Use of the engineered cell of any one of claims 1-23 or the pharmaceutical composition of claim 24 in the preparation of a medicament for preventing or treating cancer, infection or autoimmune diseases.
